(19) Europäisches Patentamt
European Patent Office
Office européen des brevets

(11) EP 4 644 523 A1

(12) **EUROPÄISCHE PATENTANMELDUNG**

(43) Veröffentlichungstag:
05.11.2025 Patentblatt 2025/45

(21) Anmeldenummer: 24172996.1

(22) Anmeldetag: 29.04.2024

(51) Internationale Patentklassifikation (IPC):
*C12M 1/00* (2006.01)  *C12M 1/36* (2006.01)

(52) Gemeinsame Patentklassifikation (CPC):
C12M 41/48; C12M 41/14

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Eppendorf SE**
**22339 Hamburg (DE)**

(72) Erfinder:
• **Schünemann, Patrick**
**Hamburg (DE)**
• **Wente, Wolf**
**22299 Hamburg (DE)**

(74) Vertreter: **Wallinger Ricker Schlotter Tostmann Patent- und Rechtsanwälte Partnerschaft mbB Zweibrückenstraße 5-7 80331 München (DE)**

(54) **STEUEREINRICHTUNG ZUR ELEKTRONISCHEN STEUERUNG EINER VERDAMPFERVORRICHTUNG EINES INKUBATORS UND VERFAHREN**

(57) Die Erfindung betrifft eine Steuereinrichtung zur Einstellung der Luftfeuchte in einer Inkubatorkammer eines inkubationsfähigen Laborgeräts, und ein Verfahren zur Einstellung der Luftfeuchte. Insbesondere steuert die Steuereinrichtung eine Luftfeuchteregeleinrichtung, eine Heizregeleinrichtung und eine Wasserfördersteuereinrichtung an.

Fig. 4

EP 4 644 523 A1

**Beschreibung**

Die Erfindung betrifft eine Steuereinrichtung zur elektronischen Steuerung eines Verdampfers eines Laborgeräts mit Inkubationsfunktion, insbesondere von Inkubatoren und Inkubationsschüttlern, ein Laborgerät mit Inkubationsfunktion für das Wachstum von biologischen Zellen und eine Verdampfervorrichtung, welche diese Steuereinrichtung aufweist, sowie ein Verfahren zur elektronischen Steuerung der Luftbefeuchtung einer Inkubatoratmosphäre.

Mit solchen inkubationsfähigen Laborgeräten, insbesondere Inkubatoren und Inkubationsschüttlern (Shaker), werden in biologischen und medizinischen Laboratorien Zellen in Zellkultur unter kontrollierten Umgebungsbedingungen gehalten, und so das Wachstum lebender Zellen in vitro oder von Bakterienkulturen ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen $CO_2$-Inkubatoren. Die Atmosphäre wird durch Luft mit einem bestimmten $CO_2$- und $O_2$-Gehalt und einer bestimmten Luftfeuchtigkeit gebildet, eine geeignete Temperatur ist oftmals 37 °C.

Die relative Luftfeuchtigkeit in der Kammer eines solchen Laborgeräts mit Inkubationsfunktion soll möglichst präzise auf einen vom Endanwender vorzugebenden Sollwert geregelt werden. Hierbei ist es wünschenswert, dass einerseits nach einer Türöffnung die Luftfeuchtigkeit möglichst schnell und dennoch ohne signifikante Überschwinger, welche zu Kondensation führen könnten, eingeregelt wird. Zusätzlich dazu soll es auch möglich sein, die bei solchen Geräten übliche, konstante Leckage der Feuchtigkeit aus der Inkubationskammer auszugleichen. Technisch herausfordernd ist dabei, dass schon sehr kleine Tropfen, die verdampfen, einen messbaren Einfluss auf die Luftfeuchtigkeit in der Inkubationskammer des Laborgeräts haben.

Bekannte Lösungen zur Regelung der Luftfeuchtigkeit bedienen sich z.B. einer Wasserwanne, die ständig innerhalb der Inkubationskammer aufbewahrt wird und daher ein latentes Risiko für Kontamination birgt, oder eines Verdampfers. Die Herausforderung bei Verwendung eines Verdampfers gegenüber einer Wasserwanne kann sein, dass ein Verdampfer zusätzliche Sensorik erfordert, um zu erkennen, ob sich Wasser im Verdampfer befindet und wie hoch die aktuelle Durchflussmenge ist. Jegliche zusätzliche Sensorik erhöht die Herstellkosten und Fertigungskomplexität des Befeuchtungssystems. Ist ein Verdampfer nicht präzise regelbar kann die Luftfeuchtigkeit in der Kammer über dem Sollwert landen. Die überschüssige Luftfeuchtigkeit muss dann z.B. mit einer zusätzlichen Pumpe, die bspw. Umgebungsluft in die Kammer fördert, wieder abgeführt werden. Dies bedeutet wiederum eine Steigerung der Kosten und Komplexität des Gesamtsystems.

Aufgabe der Erfindung ist es daher, eine Steuereinrichtung für ein Laborgerät mit Verdampfervorrichtung und ein Verfahren zur Steuerung anzugeben, welche eine effiziente Regelung der Luftfeuchtigkeit bewirken.

Die Erfindung löst diese Aufgabe durch die Steuereinrichtung gemäß Anspruch 1 und das Verfahren gemäß Anspruch 14. Bevorzugte Ausgestaltungen der Erfindung ergeben sich insbesondere aus den Unteransprüchen und der nachfolgenden Beschreibung der Erfindung.

Die Erfindung betrifft eine

Steuereinrichtung zur elektronischen Einstellung der Luftfeuchte einer Inkubatoratmosphäre eines Inkubators zur Inkubation lebender Zellkulturen mittels einer Verdampfervorrichtung, aufweisend

- eine Luftfeuchteregeleinrichtung, die dazu eingerichtet ist, den Luftfeuchtewert auf einen Luftfeuchtesollwert zu regeln,
- eine Heizregeleinrichtung, die dazu eingerichtet ist, die mittels eines Temperatursensors messbare Temperatur eines wasserverdampfenden Heizelements der Verdampfervorrichtung auf eine konstante Zieltemperatur zu regeln, und dabei als Stellgröße einen Verdampfungswert zu verwenden,

  und wobei sich insbesondere der Verdampfungswert aus einer Messung einer bei dieser Regelung auftretenden Heizleistung oder einer Temperaturabweichung der gemessenen Temperatur von der Zieltemperatur ergibt, wobei insbesondere der Verdampfungswert durch ein Wasservolumen bestimmt wird, das vom Heizelement verdampft wird, und wobei die Zieltemperatur als Verdampfungstemperatur wählbar ist, die geeignet ist, ein mit dem Heizelement kontaktiertes Wasservolumen zu verdampfen;

- eine Wasserfördersteuereinrichtung, die dazu eingerichtet ist, einen Wasserförderleistungsparameter zur Ansteuerung einer Wasserfördervorrichtung zu verwenden, der ein aktuell zu förderndes Wasservolumen bestimmt,

- wobei die Steuereinrichtung dazu eingerichtet ist, den Wasserförderleistungsparameter in Abhängigkeit von dem Verdampfungswert zu variieren, bis ein mittels Luftfeuchtesensor messbarer Luftfeuchtewert der Inkubatoratmosphäre einem Luftfeuchtesollwert entspricht.

Die Erfindung gemäß bevorzugter Ausführungsform ermöglicht eine robuste und präzise Regelung der Luftfeuchtigkeit in allen Betriebszuständen eines Laborgeräts zur Inkubation, insbesondere eines Inkubationsschüttlers oder Inkubators.

Die Steuereinrichtung bzw. deren Programmcode bzw. Algorithmus überwacht eine Leistung, insbesondere mittlere Leistung des Heizelements, die indirekt ermittelt wird, zum Beispiel durch den aktuellen Stellwert des Heizelements, insbesondere über ein Tastverhältnis im Falle einer PWM-Steuerung des Heizelements, oder der Abweichung der Temperatur des Heizelements vom Temperatursollwert. Die Steuereinrichtung kann so feststellen, ob aktuell Wasser verdampft wird, und insbesondere auch das verdampfte Wasservolumen quantifizieren. Die genannte Leistung des Heizelements bzw. dessen Stellwert wird deshalb vorliegend auch als Wasservolumenverdampfungsleistungswert, kurz "Verdampfungswert", bezeichnet.

Über die programmcode-gesteuerte Ansteuerung der Wasserfördereinrichtung, insbesondere Pumpeinrichtung, ist es möglich die verdampfe Wassermenge zu steuern bzw. zu regeln. Der Stellwert, der nötig ist, um das Heizelement auf der Zieltemperatur von z.B. 180 °C zu halten, ist direkt abhängig von der Heizelementleistung, insbesondere direkt proportional zu der Leistung, die durch die Verdampfung am Heizelement in Wärme umgewandelt wird. Die nötige Leistung wiederum ist direkt proportional zu der Menge an Wasser, die im Mittel der Verdampfervorrichtung zugeführt wird.

Insbesondere das schnelle Einregeln ohne signifikante Überschwinger sowie das Nachführen kleinster Wassermengen im statischen Zustand sind Vorteile der Erfindung. Dies wird dadurch erreicht, dass die Steuereinrichtung der Verdampfervorrichtung nur immer so viel Wasser zuführt, wie innerhalb kürzester Zeit verdampft werden kann. Dies wird durch die Einbeziehung des aktuell anliegenden Verdampfungswertes, z.B. der Heizleistung, in die Steuerung bzw. Regelung der Wasserförderleistung der Wasserfördereinrichtung bewerkstelligt. Wird kein Dampf mehr benötigt wird die Wasserfördereinrichtung abgestellt und es verbleibt keine größere Menge Wasser innerhalb des Verdampfers, die zu einem längeren Nachlaufverhalten führen würde.

Überdies benötigt die Steuereinrichtung keinen Füllstandsensor in einem Wassertank, da durch eine, über einen gewissen Zeitraum zu niedrig angeforderte, Heizleistung am Heizelement festgestellt werden kann, dass kein Wasser im Verdampfer ankommt. Ein weiterer Vorteil ist, dass kein Durchflusssensor im Zuführungsschlauch oder am Verdampfer selbst nötig ist, was zu Einsparungen bei den Herstellkosten führt. Dies wird durch die Quantifizierung des verdampften Wasservolumens mittels des genannten Verdampfungswertes ermöglicht.

Der Begriff "Steuerungseinrichtung" bezieht sich auf eine Systemsteuerung, die die gewünschte Steuerung der Luftfeuchte, hier die Regelung der Luftfeuchte, durch Maßnahmen der Regelung und der Steuerung erreicht.

In einem engen Sinne bezieht sich Steuerung auf den Prozess, bei dem eine oder mehrere Eingangsgrößen eines Systems verwendet werden, um eine Prozessgröße zu beeinflussen. Der tatsächliche Wert der Prozessgröße wird nicht überwacht, was bedeutet, dass Abweichungen, die beispielsweise durch externe Störungen verursacht werden, den Steuerungsprozess nicht beeinflussen. Ein offener Wirkungsablauf kennzeichnet somit die Steuerung im engen Sinne.

Im Gegensatz dazu wird bei einer Regelung - vorzugsweise kontinuierlich, aber auch diskontinuierlich - die zu regulierende Größe (Regelgröße x) gemessen und mit einem vorgegebenen Wert (Führungsgröße, Sollwert w) verglichen. Wenn eine Differenz zwischen diesen Größen besteht (Regeldifferenz e bzw. Regelabweichung x-w), wird je nach gemessener Differenz ein Anpassungsprozess eingeleitet, um die Regelgröße wieder mit der Führungsgröße in Einklang zu bringen. Ein geschlossener Wirkungsablauf kennzeichnet somit die Regelung.

In DIN 19 226 ist der Begriff der Regelung so definiert: "Das Regeln, die Regelung, ist ein Vorgang, bei dem fortlaufend eine Größe, die Regelgröße (zu regelnde Größe), erfasst, mit einer anderen Größe, der Führungsgröße, verglichen und im Sinne einer Angleichung an die Führungsgröße beeinflusst wird. Kennzeichen für das Regeln ist der geschlossene Wirkungsablauf, bei dem die Regelgröße im Wirkungsweg des Regelkreises fortlaufend sich selbst beeinflusst."

Als "fortlaufend" gilt hier auch eine hinreichend häufige Wiederholung gemäß einer Anzahl N von gleichartigen Einzelvorgängen. Möglich wäre im Kontext des zweiten Betriebsmodus der Steuerungseinrichtung aber grundsätzlich auch bereits N=1, also ein einzelnes Feedback, die auch bereits einen dynamischen Vorgang kennzeichnet, insofern die zu regelnde Luftfeuchte dann bereits erreicht wird.

Die Steuerungseinrichtung (auch bezeichnet als "Steuereinrichtung") weist eine Luftfeuchteregelungseinrichtung auf, eine Heizregeleinrichtung und eine Wasserfördersteuereinrichtung. Letztere ist nur optional als Regelung (Wasserförderregeleinrichtung) ausgebildet und/oder weist eine solche auf.

Die Steuerungseinrichtung ist ein elektronisches, insbesondere datenverarbeitendes Gerät, das insbesondere elektrisch verbunden ist mit den Messsensoren der Regelungen, insbesondere mit dem Luftfeuchtesensor in der Inkubationskammer, dem Heizelementtemperatursensor, einer Verdampfungswert-Signalausgabe der Heizelementregelung) und mit einem oder mehr Stellgliedern der Regelungen, insbesondere einem Eingang der Wasserfördereinrichtung, insbesondere Pumpeinrichtung, zur Festlegung der Wasserförderleistung (Pumpleistung).

Deshalb weist die Steuerungseinrichtung vorzugsweise eine Prozessoreinrichtung mit Datenspeicher auf, insbesondere einen Mikrocontroller, der dazu programmiert ist, die Funktionen der Luftfeuchteregelungseinrichtung auf, eine Heizregeleinrichtung und eine Wasserfördersteuereinrichtung, insbesondere Wasserförderregeleinrichtung, zu implementieren. Es ist aber auch möglich und bevorzugt, dass die Steuerungseinrichtung mehr als eine Prozessoreinrichtung mit Datenspeicher aufweist, insbesondere dass jede der Luftfeuchteregeleinrichtung, Heizregeleinrichtung, Wasserfördersteuer-/regeleinrichtung eine eigene oder gemeinsame Prozessoreinrichtung mit Datenspeicher aufweist.

Die Heizregeleinrichtung arbeitet vorzugsweise autonom, d.h. die Steuerungseinrichtung ist nicht dazu eingerichtet, dass der Stellwert der Heizregeleinrichtung durch Signale von außerhalb der Heizregeleinrichtung beeinflusst wird: die Heizregeleinrichtung regelt grundsätzlich die Heizelementtemperatur immer selbständig und unabhängig auf den gesetzten Sollwert, insbesondere den Verdampfungssollwert (z.B. 180 °C).

Vorzugsweise ist die Steuereinrichtung dazu eingerichtet, in einem ersten Betriebsmodus betrieben zu werden und/oder in einem zweiten Betriebsmodus betrieben zu werden. Ein Betriebsmodus ist insbesondere durch ein Betriebsmodus-spezifisches Verfahren zur Steuerung / Regelung der Luftfeuchte gekennzeichnet, wobei dieses Verfahren mittels der Steuerungseinrichtung implementiert ist, die in geeigneter Weise elektronisch eingerichtet und/oder programmiert ist, das Verfahren auszuführen.

Vorzugsweise erfolgt im ersten Betriebsmodus die Einstellung des Wasserförderleistungsparameters durch eine Wasserförderregelung. Dies ermöglicht eine kontinuierliche Dampfproduktion mit insbesondere hoher Produktionsrate.

Vorzugsweise erfolgt im zweiten Betriebsmodus die Einstellung des Wasserförderleistungsparameters nicht reguliert, insbesondere, indem nicht kontinuierlich, insbesondere auch nicht in Einzelschritten oder diskontinuierlich - die zu regulierende Größe (Regelgröße x, hier: Wasserförderleistungsparameter) gemessen und mit einem vorgegebenen Wert (Führungsgröße, Sollwert w, hier Sollwert für Wasserförderleistungsparameter) verglichen wird. Insbesondere wird dann die Wasserfördereinrichtung so angesteuert, dass insbesondere eine Feinregulierung des Luftfeuchtewertes erreicht wird, mit insbesondere niedriger Dampfproduktionsrate.

Vorzugsweise ist die Steuereinrichtung dazu eingerichtet, insbesondere programmiert, in dem zweiten Betriebsmodus betrieben zu werden, wenn der Luftfeuchtewert einen ersten Schwellwert unterschreitet, und in dem ersten Betriebsmodus betrieben zu werden, wenn der Luftfeuchtewert einen zweiten Schwellwert unterschreitet, wobei der zweite Schwellwert niedriger ist als der erste Schwellwert,

wobei insbesondere der erste Schwellwert in einem Bereich von vorzugsweise 0,01-0,4%, vorzugsweise 0,5 - 0,3%, vorzugsweise 0,1 - 0,25%, vorzugsweise 0,15 - 0,25% (gemeint ist jeweils Prozent relative Luftfeuchte) unter dem Luftfeuchtesollwert liegt, also zum Beispiel als erster Schwellwert x: 94,8% relative Luftfeuchte bei einem Luftfeuchtesollwert von 95% relative Luftfeuchte mit x=0,2%;

wobei insbesondere der der zweite Schwellwert in einem Bereich von vorzugsweise 0,4% bis 3,0%, vorzugsweise 1,0 - 2,5%, unter dem Luftfeuchtesollwert liegt, also zum Beispiel als zweiter Schwellwert y: 93,5% relative Luftfeuchte bei einem Luftfeuchtesollwert von 95% relative Luftfeuchte mit y=1,5%.

Der erste Schwellwert x für den Wechsel vom zweiten zum ersten Betriebsmodus ist vorzugsweise ausgewählt aus dem Bereich mit bevorzugten Werten {0,1%;0,5%}. Der zweite Schwellwert y für den Wechsel vom zweiten zum ersten Betriebsmodus ist vorzugsweise ausgewählt aus dem Bereich mit bevorzugten Werten {0,5%;3,5%}

Die Bereitstellung von zwei Betriebsmodi, wie beschrieben, ist ein besonderer Vorteil, da von der Steuereinrichtung automatisch flexibel auf Luftfeuchteverluste reagiert werden kann. Beispielsweise setzt im Falle höherer Luftfeuchteverluste bei plötzlich gewählten höheren Drehzahlen der Schüttelbewegung automatisch der erste Betriebsmodus mit höherer Dampfproduktionsrate ein.

Vorzugsweise weist die Steuereinrichtung eine Luftzuführungssteuereinrichtung auf, die in einem weiteren Betriebsmodus, der z.B. als dritter Betriebsmodus bezeichnet werden kann, der Steuereinrichtung angesteuert wird, insbesondere um die Luftfeuchte in der Kammer zu senken.

Der zweite Betriebsmodus ist insbesondere geeignet, kleinere Luftfeuchteverluste (Schwellwert x) in der Kammer zu korrigieren, die typischer Weise im längerfristigen Betrieb der geschlossenen Kammer aufgrund von Undichtigkeiten auftreten können. Im normalen Betrieb des Laborgeräts mit geschlossener Kammer ist es sehr unwahrscheinlich oder unmöglich, dass größere Luftfeuchteverluste (Schwellwert y) auftreten, die die Steuereinrichtung zur Ausführung des ersten Betriebsmodus veranlassen würden.

Der erste Betriebsmodus wird in der Praxis in solchen Situationen aktiviert werden, in denen ein plötzlicher Luftfeuchteabfall in der Kammer erfolgt ist, wie dies typischer Weise nach einer Phase mit geöffneter Türe der Kammer der Fall ist. Diese Phase tritt ein, wenn ein Benutzer den Kammerinnenraum neu bestückt, inspiziert, oder Probengefäße entnimmt. Mit dem Schließen der Türe endet diese Phase, und die Steuereinrichtung ermittelt die Luftfeuchte in der Kammer, und wählt entsprechend den passenden Betriebsmodus. Arbeitet die Steuereinrichtung im ersten Betriebsmodus, wird die Luftfeuchte der Kammer in diesem ersten Betriebsmodus auf den Luftfeuchtesollwert w_rH zurückgeführt, insbesondere ohne dass ein Wechsel in den zweiten Betriebsmodus notwendig ist oder erfolgt. Dies wäre aber grundsätzlich möglich, insbesondere beim Erreichen eines Schwellwertes, der z.B. w_rH - 2*x betragen kann. Ist (im ersten oder zweiten Betriebsmodus) die Luftfeuchte wieder auf den Luftfeuchtesollwert geregelt, endet der jeweilige Betriebsmodus.

In einem vorzugsweise vorgesehenen weiteren Betriebsmodus, der z.B. als dritter Betriebsmodus bezeichnet werden kann, wird die Zieltemperatur des Heizelements der Verdampfervorrichtung gesenkt, insbesondere um 10-75%, z.B. von 180°C auf 90°C, oder derart, dass die Zieltemperatur der Temperatur des Kammerinnenraums entspricht. Dadurch wird die Verdampfervorrichtung geschont und ein unnötiger Wärmeeintrag der Verdampfervorrichtung in das Laborgerät reduziert oder vermieden, der insbesondere die Temperatur des Kammerinnenraums ungewollt beeinflussen könnte.

Vorzugsweise weist die Steuereinrichtung einen in einem Programmcodespeicher der Steuereinrichtung gespeicherten Programmcode auf, durch dessen Ausführung ein Verdampfungsvorgang durch Definieren des Wasserförderleistungsparameters gestartet, gestoppt oder beibehalten wird.

Vorzugsweise ist die Steuereinrichtung dazu eingerichtet, insbesondere programmiert, als Verdampfungswert eine mittlere Leistung des Heizelements zu verwenden und zu überwachen, und dadurch insbesondere Information in Form von Daten zu gewinnen und insbesondere in einer Datenspeichereinrichtung der Steuereinrichtung zu speichern, insbesondere Informationen darüber, ob aktuell Wasser verdampft wird, insbesondere entsprechend einem zum Verdampfungswert korreliertem, insbesondere proportionalen, Wasservolumen, oder ob kein Wasser verdampft wird.

Vorzugsweise ist die Steuereinrichtung dazu eingerichtet, insbesondere programmiert, den Luftfeuchtewert zu regeln, indem der Luftfeuchtewert dem Luftfeuchtesollwert asangenähert wird, ohne dass der Luftfeuchtesollwert überschritten wird, insbesondere durch asymptotische Annäherung an den Sollwert, so dass insbesondere ein Überschwingen verhinderbar ist. Dies wird insbesondere dadurch erreicht, dass die verwendete PI-Regelung des Luftfeuchteegelkreises so parametriert ist, dass je näher sich der Ist-Wert (x_rH) dem Ziel-Wert (w_rH) nähert, die Dampfproduktion reduziert wird. Dies führt zu einem immer langsameren Steigungsverhalten des rH-Wertes (x_rH).

Erster Betriebsmodus

Vorzugsweise ist die Steuereinrichtung dazu eingerichtet, insbesondere programmiert, dass im ersten Betriebsmodus der Verdampfungswert zur Bestimmung der Regelgröße (x_WP) einer Wasserförderregelung verwendet wird. Der Verdampfungswert gibt eine präzise Information über das tatsächlich aktuell verdampfte Wasservolumen, und eignet sich deshalb besonders zur Regelung der Wasserförderung.

Vorzugsweise ist die Steuereinrichtung dazu eingerichtet, insbesondere programmiert, dass im ersten Betriebsmodus ein Verdampfungssollwert (w_WP) als Stellgröße der Wasserförderregelung verwendet wird. Die Dampfmenge, die die Verdampfervorrichtung produziert, wird dann dadurch bestimmt, wie viel Leistung das Heizelement verbrauchen soll. Diese Zielgröße wird durch die Regelung der Wasserförderleistung eingehalten. Dadurch entsteht der Vorteil, dass kein Durchflusssensor im Zuführungsschlauch zur Verdampfervorrichtung oder an der Verdampfervorrichtung selbst nötig ist, was zu Einsparungen bei den Herstellkosten führt.

Der Verdampfungssollwert bestimmt insbesondere ein Wasservolumen, dessen Verdampfung zur Einstellung der Luftfeuchte der Inkubatoratmosphäre erforderlich ist, wobei insbesondere der Luftfeuchtesollwert vom Inkubator bzw. dem Benutzer vorgegeben wird. Insbesondere ist in einem dritten Betriebsmodus auch möglich, dass der Luftfeuchtewert x_rH auf einen vom Benutzer oder anders vorgegebenen Luftfeuchtesollwert w_rH geregelt wird, wobei w_rH insbesondere geringer sein kann als der aktuelle gemessene Luftfeuchtewert x_rH, dass also die Kammeratmosphäre entfeuchtet werden muss. Dazu dient insbesondere die Luftzuführungssteuereinrichtung zur Steuerung einer Luftzuführungseinrichtung, insbesondere einer Luftpumpe, des Laborgeräts.

Vorzugsweise weist die Wasserfördersteuereinrichtung eine Wasserförderregeleinrichtung auf, die dazu eingerichtet ist, in dem ersten Betriebsmodus der Steuereinrichtung den Verdampfungswert auf den Verdampfungssollwert zu regeln, und dabei als Stellgröße den Wasserförderleistungsparameter zu verwenden.

Eine Steuereinrichtung zur elektronischen Steuerung der Befeuchtung der Inkubatoratmosphäre eines Inkubators zur Inkubation lebender Zellkulturen mittels einer Verdampfervorrichtung, insbesondere für eine kontinuierliche Dampfproduktion, weist vorzugsweise auf:

- eine Luftfeuchteregeleinrichtung, die dazu eingerichtet ist, einen mittels Luftfeuchtesensor messbaren Luftfeuchtewert der Inkubatoratmosphäre auf einen vom Inkubator bzw. dem Benutzer vorgegebenen Luftfeuchtesollwert zu regeln, und dabei als Stellgröße einen Verdampfungssollwert zu verwenden, wobei insbesondere dieser Verdampfungssollwert zu einem Wasservolumen proportional ist, dessen Verdampfung zur Einstellung der Luftfeuchte der Inkubatoratmosphäre erforderlich ist,

- eine Wasserförderregeleinrichtung, die dazu eingerichtet ist, einen Verdampfungswert auf den Verdampfungssollwert zu regeln, und dabei als Stellgröße einen Wasserförderleistungsparameter einer Wasserfördervorrichtung zu verwenden,

wobei insbesondere dieser Verdampfungswert zu einem aktuell verdampften Wasservolumen proportional ist und dieser Wasserförderleistungsparameter zu einem aktuell geförderten Wasservolumen proportional ist,

- eine Heizregeleinrichtung, die dazu eingerichtet ist, die mittels eines Temperatursensors messbare Temperatur eines Heizelements der Verdampfervorrichtung auf eine konstante Zieltemperatur zu regeln, und dabei als Stellgröße den Verdampfungswert zu verwenden,

wobei insbesondere die Zieltemperatur geeignet ist, ein bei einem Verdampfungsvorgang mit dem Heizelement in Kontakt kommendes Wasservolumen zu verdampfen und dabei dem Heizelement Wärme zu entziehen, und wobei insbesondere der Verdampfungswert zu einem Wasservolumen proportional ist, das vom Heizelement verdampft wird, und der sich aus einer Messung einer bei dieser Regelung auftretenden Heizleistung oder einer Temperaturabweichung der gemessenen Temperatur von der Zieltemperatur ergibt.

Vorzugsweise ist die Steuereinrichtung dazu eingerichtet, insbesondere programmiert" den von einem Türsensor eines Inkubators erfassten Türöffnungswert zu erfassen und zu verarbeiten, und insbesondere die Wasserfördereinrichtung abzuschalten und/oder die Leistung des Heizelements zu reduzieren, wenn gemäß dem Türöffnungswert eine Türe der Inkubatorkammer des Inkubators geöffnet ist, und/oder, wenn gemäß dem Türöffnungswert eine Türe der Inkubatorkammer des Inkubators nach einer Türöffnung wieder geschlossen ist, in einem ersten Betriebsmodus betrieben zu werden, und insbesondere in dem ersten Betriebsmodus die Wasserfördereinrichtung kontinuierlich zu betreiben.

Zweiter Betriebsmodus

Vorzugsweise ist die Steuereinrichtung dazu eingerichtet, insbesondere programmiert, dass im zweiten Betriebsmodus der Wasserförderleistungsparameter (WP) als Stellgröße (y_rH) der Luftfeuchteregelung verwendet wird. Dies ermöglicht eine direkte Festlegung der zu verdampfenden Wassermenge, insbesondere ohne den Umweg über eine Regelungsschleife, und erlaubt somit eine Feinsteuerung der Wasserfördereinrichtung, insbesondere im Volumenbereich zwischen 2 $\mu$l und 15 $\mu$l. Dieses Volumen entspricht einem relativ kleinen Wassertropfen, das zum Heizelement gelangt. Es kann über die gewünschte Pumpleistung festgelegt werden.

Vorzugsweise ist die Steuereinrichtung dazu eingerichtet, insbesondere programmiert, dass die Verdampfung eines Tropfens bzw. die Verdampfung eines dem Tropfen entsprechenden Wasservolumens, insbesondere im Volumenbereich zwischen 2 $\mu$l und 15 $\mu$l, detektiert wird, insbesondere durch die Erfassung und insbesondere Auswertung des Verdampfungswertes (y_TC).

Vorzugsweise ist die Steuereinrichtung dazu eingerichtet, insbesondere programmiert, dass im zweiten Betriebsmodus der Wasserförderleistungsparameter (WP) insbesondere von der Steuereinrichtung in Abhängigkeit von dem Verdampfungswert (y_TC) eingestellt wird.

Vorzugsweise ist die Steuereinrichtung dazu eingerichtet, insbesondere programmiert, dass im zweiten Betriebsmodus über eine zeitabhängige Erfassung und Verarbeitung des Verdampfungswertes, der insbesondere durch ein Temperaturabfall oder eine Heizleistungserhöhung des Heizelements definiert ist, zu ermitteln, ob ein vorgegebener Schwellwert des Verdampfungswertes überschritten wurde, insbesondere innerhalb einer vorbestimmten Zeitdifferenz. Die mittels Schwellwert zuverlässig detektierbare Änderung des Verdampfungswertes ist indikativ für ein Wasservolumen, das am Heizelement eintrifft, diesem Wärme entzieht und somit eine Erhöhung der Heizleistung triggert, welche zur Nachregelung der Heizelementtemperatur auf die gewünschte Solltemperatur erforderlich ist. Insbesondere ist der Schwellwert so gewählt, dass sein Überschreiten ein Kontaktieren der Heizfläche durch ein bestimmtes Wasservolumen (z.B.: 2 $\mu$l - 15 $\mu$l) anzeigt. Dieser Schwellwert kann zur vollständigen Definition des Steuerungsverfahrens herstellerseitig ermittelt werden. Der Schwellwert ist dann insbesondere in einer Datenspeichereinrichtung der Steuerungseinrichtung gespeichert und von dort durch das Steuerungsverfahren bzw. die Steuerungseinrichtung abrufbar.

Vorzugsweise ist die Steuereinrichtung dazu eingerichtet, insbesondere programmiert, dass im zweiten Betriebsmodus zunächst ein Wasservolumeninkrement von zwischen z.B. 2 $\mu$l - 15 $\mu$l gefördert wird, insbesondere dass dann unmittelbar nachfolgend in einem vorbestimmten Zeitraum der Verdampfungswert mit dem Schwellwert wiederholt verglichen wird, dann insbesondere im Falle eines Überschreitens des Schwellwertes für eine vorbestimmte Zeitspanne der Luftfeuchtewert beobachtet und mit mindestens einem, zuvor ermittelten und in einer Datenspeichereinrichtung gespeicherten Luftfeuchtewert, verglichen wird. Ergibt sich ein Ansteigen des Luftfeuchtewertes, dann war die Wasserförderung offenbar erfolgreich. Es wird dann vorzugsweise abgewartet, ob der Luftfeuchtewert auf den Luftfeuchtesollwert zuläuft, insbesondere ohne den Luftfeuchtesollwert zu überschreiten, insbesondere durch asymptotische Annäherung. Ist dies der Fall, so ist die Nachregelung erfolgreich beendet. Ist dies nicht der Fall, wird erneut ein Wasservolumeninkrement von zwischen z.B. 2 $\mu$l - 15 $\mu$l gefördert, u.s.w..

Während der genannten Zeitspanne wird insbesondere die Wasserfördereinrichtung so gesteuert, dass keine Wasserförderung erfolgt, insbesondere wird in dieser Zeitspanne der Wasserförderleistungsparameter (WP) auf Null gesetzt - die Wasserfördereinrichtung ist dann deaktiviert.

Vorzugsweise ist die Steuereinrichtung dazu eingerichtet, insbesondere programmiert, dass die Wasserfördereinrichtung abgeschaltet wird, wenn festgestellt wird, dass der vorgegebene Schwellwert des Verdampfungswertes innerhalb der vorbestimmten Zeitdifferenz überschritten wurde.

Vorzugsweise ist die Steuereinrichtung dazu eingerichtet, insbesondere programmiert, dass, festzustellen, ob der Verdampfungssollwert innerhalb einer vorgegebenen Zeitspanne erreicht wurde, und insbesondere, falls dies nicht der Fall ist, die Wasserfördereinrichtung abzuschalten, insbesondere um ein Betreiben der Pumpeneinrichtung bei einem ungefüllten Zustand des zuführenden Wasserschlauches oder des Wasserreservoirs zu verhindern.

Vorzugsweise ist die Steuereinrichtung dazu eingerichtet, insbesondere programmiert, in dem ersten Betriebsmodus die Wasserfördereinrichtung, insbesondere überwiegend, kontinuierlich zu betreiben, und insbesondere in dem zweiten Betriebsmodus die Wasserfördereinrichtung, insbesondere überwiegend, diskontinuierlich zu betreiben.

Vorzugsweise ist die Steuereinrichtung dazu eingerichtet, insbesondere programmiert, in dem zweiten Betriebsmodus die Wasserfördereinrichtung intermittierend zu betreiben. Intermittierend bedeutet, dass ein im Falle des kontinuierlichen Betrieb anliegendes Steuersignal unterbrochen wird. Im Falle eines mittels Pulsweitenmodulation (PWM) modulierten Eingangssignals einer Wasserfördereinrichtung wird das Eingangssignal dann abwechselnd auf Null gesetzt (Unterbrechungsphase) und auf einen von Null verschiedenen Betriebswert gesetzt (Betriebsphase).

Vorzugsweise erfordert ein kontinuierlicher Betrieb der Wasserfördereinrichtung, dass die Wasserfördereinrichtung für eine Mindestdauer t_min2 ohne Unterbrechung angesteuert wird, während der insbesondere der Wasserförderleistungsparameter größer ist als Null, wobei vorzugsweise 5 s <= t_min2 <= 5000 s.

Vorzugsweise erfordert der kontinuierliche Betrieb der Wasserfördereinrichtung, dass die Wasserfördereinrichtung für eine Mindestdauer t_min2 ohne Unterbrechung angesteuert wird, während der insbesondere der Wasserförderleistungsparameter größer ist als Null, wobei vorzugsweise y1 <= t_min2 <= y2, wobei vorzugsweise y1 gewählt ist aus den jeweils bevorzugten Werten {5,0 s; 10,0 s; 20 s; 30 s; 60 s}, wobei y2 gewählt ist aus den jeweils bevorzugten Werten {60 s; 120 s; 500 s; 1000 s; 5000 s; 10000 s},

Vorzugsweise erfordert ein diskontinuierlicher Betrieb der Wasserfördereinrichtung, dass die Wasserfördereinrichtung mit Unterbrechungen angesteuert wird und sequenziell mit einer Maximaldauer t_max1 angesteuert wird, wobei während der Unterbrechungen insbesondere der Wasserförderleistungsparameter Null beträgt, und die Unterbrechungen eine Mindestdauer t_min1 aufweisen, wobei vorzugsweise 0,01 s <= t_max1 <= 5,0 s, und wobei insbesondere 5,0 s <= t_min1 <= 200 s.

Vorzugsweise erfordert der diskontinuierliche Betrieb der Wasserfördereinrichtung, dass die Wasserfördereinrichtung mit Unterbrechungen angesteuert wird und sequentiell mit einer Maximaldauer t_max1 angesteuert wird, wobei während der Unterbrechungen insbesondere der Wasserförderleistungsparameter Null beträgt, und die Unterbrechungen eine Mindestdauer t_min1 aufweisen, wobei vorzugsweise x1 <= t_max1 <= x2, und wobei insbesondere x3 <= t_min1 <= x4, wobei x1 gewählt ist aus den jeweils bevorzugten Werten {0,01 s; 0,1 s; 0,5 s; 1,0 s}, wobei x2 gewählt ist aus den jeweils bevorzugten Werten {0,5 s; 1,0 s; 2,0 s; 5,0 s}, wobei x3 gewählt ist aus den jeweils bevorzugten Werten {5 s; 10 s; 20 s}, wobei x4 gewählt ist aus den jeweils bevorzugten Werten {30 s; 50 s; 100 s; 200 s}.

Vorzugsweise ist die Steuereinrichtung dazu eingerichtet, insbesondere programmiert, in dem zweiten Betriebsmodus die Wasserfördereinrichtung intermittierend zu betreiben, die Wasserfördereinrichtung diskontinuierlich zu betreiben, insbesondere durch eine sequentielle Förderung einer definierten Anzahl M (M = 1, 2, 3, ...) von Volumeninkrementen, die jeweils vorzugsweise zwischen 2 µl und 20 µl betragen, vorzugsweise zwischen 5 µl und 15 µl.

Vorzugsweise ist die Steuereinrichtung dazu eingerichtet, insbesondere programmiert, in dem zweiten Betriebsmodus die Wasserfördereinrichtung intermittierend zu betreiben, eine Anzahl M (M = 1, 2, 3, ...) von einzelnen Volumeninkrementen zu fördern, wobei die Steuerung der Leistung der Wasserfördereinrichtung insbesondere durch Vorgabe einer Wasserförderfrequenz, gemessen in Volumeninkrementen pro Minute (M/Minute), erfolgt.

Vorzugsweise ist die Steuereinrichtung dazu eingerichtet, insbesondere programmiert, in dem zweiten Betriebsmodus die Wasserfördereinrichtung in einem Pulsbetrieb zu betreiben, bei dem die einzelnen Betriebspulse eine Dauer von kleiner als vorzugsweise 3s, vorzugsweise 2s, vorzugsweise 1 s haben, wobei die Dauer vorzugsweise geeignet ist, mittels der Wasserfördereinrichtung mit jedem Betriebspuls eine Volumeninkrement des Wassers zu fördern, insbesondere entsprechend einem Pumpenhub. Die Betriebspulse sind vorzugsweise getrennt durch Zeitspannen, die unterschiedlich oder gleich sein können. Die Dauer der Zeitspanne kann von der Steuerungseinrichtung in Abhängigkeit von mindestens einem anderen Parameter variiert werden, insbesondere abhängig vom Verdampfungswert und/oder dem Luftfeuchtewert.

Luftentfeuchtung

Die Erfindung betrifft auch eine Steuereinrichtung (100') zur elektronischen Einstellung der Luftfeuchte einer Inkubatoratmosphäre eines Laborgeräts (50) zur Inkubation lebender Zellkulturen mittels einer Luftentfeuchtervorrichtung (200) und einer Verdampfervorrichtung (1),

wobei die Steuereinrichtung, insbesondere deren Luftentfeuchtersteuereinrichtung (104), dazu eingerichtet, insbesondere programmiert ist, die Luftentfeuchtervorrichtung (200) zu steuern, mit der die Inkubatoratmosphäre in einer Inkubatorkammer des Laborgeräts entfeuchtbar ist, und

wobei die Steuereinrichtung, insbesondere deren Luftfeuchteregeleinrichtung (101), dazu eingerichtet, insbesondere programmiert ist, die Verdampfervorrichtung (1) zu steuern, mit der die Inkubatoratmosphäre in der Inkubatorkammer des Laborgeräts befeuchtbar ist.

Die Erfindung betrifft auch ein Laborgerät mit einer Inkubationskammer zur Inkubation lebender Zellkulturen und diese Steuereinrichtung (100'). Bevorzugte Ausgestaltungen des Laborgeräts und/oder der Steuereinrichtung sind der gesamten Beschreibung dieser Patentanmeldung entnehmbar.

Vorzugsweise ist die Steuereinrichtung dazu eingerichtet, insbesondere programmiert, die Luftentfeuchtervorrichtung und die Verdampfervorrichtung so zu steuern, dass ein Luftfeuchtewert rH im Kammerinnenraum der Inkubatorkammer ausgehend von einem aktuellen Messwert rH_start der Luftfeuchte und von einem aktuellen Messwert der Temperatur T_start im Kammerinnenraum in Abhängigkeit von einem Temperaturzielwert Tziel (Tziel ungleich T) eingestellt wird, insbesondere bevor oder während die Temperatur im Kammerinnenraum auf den Temperaturzielwert geregelt wird, wobei der Temperaturzielwert der von einem Benutzer oder ein elektronisch vorgegebener Temperatursollwert ist, auf den die Temperatur im Kammerinnenraum von einer Temperaturregelungseinrichtung der Steuereinrichtung geregelt werden soll. Die Einstellung der Luftfeuchte kann auch unmittelbar im Anschluss an die Temperaturänderung erfolgen. Durch die Vorwegnahme der Änderung der absoluten Luftfeuchte muss die Luftfeuchte nicht durch einen zeitraubenden Regelvorgang eingestellt werden, bei dem die relative Luftfeuchte auf ihren Sollwert geführt wird. Ein verlängerter Zeitraum bei einer unpassenden Luftfeuchte kann insbesondere Kondensation bewirken oder für empfindliche Proben schädlich sein. Diese Regelung der Luftfeuchte kann aber, insbesondere nach der Einstellung der Luftfeuchte, erfolgen.

Vorzugsweise ist der einzustellende Luftfeuchtewert rH_ziel der absoluten Luftfeuchte niedriger als der vom Luftfeuchtesensor gemessene Luftfeuchtewert rH_start absoluter Luftfeuchte, wenn Tziel < Tstart.

Vorzugsweise ist der einzustellende Luftfeuchtewert rH_ziel der absoluten Luftfeuchte höher als der vom Luftfeuchtesensor gemessene Luftfeuchtewert rH_start absoluter Luftfeuchte, wenn Tziel > Tstart.

Der Wert der relativen Luftfeuchte, insbesondere der Luftfeuchtewert x_rH, bleibt in der Kammer vorzugsweise unverändert, vergleicht man den Wert der relativen Luftfeuchte vor Temperaturänderung mit dem Wert nach Temperaturänderung.

Die Erfindung betrifft auch eine Luftentfeuchtersteuereinrichtung (104) zur elektronischen Einstellung der Luftfeuchte einer Inkubatoratmosphäre eines Laborgeräts (50) zur Inkubation lebender Zellkulturen mittels einer Luftentfeuchtervorrichtung (200), wobei die Luftentfeuchtersteuereinrichtung (104) dazu eingerichtet, insbesondere programmiert ist, die Luftentfeuchtervorrichtung (200) zu steuern, mit der die Inkubatoratmosphäre in einer Inkubatorkammer des Laborgeräts entfeuchtbar ist. Die Erfindung betrifft auch ein Laborgerät mit einer Inkubationskammer zur Inkubation lebender Zellkulturen und diese Luftentfeuchtersteuereinrichtung (104). Bevorzugte Ausgestaltungen des Laborgeräts sind der gesamten Beschreibung dieser Patentanmeldung entnehmbar.

Die Luftentfeuchtersteuereinrichtung kann insbesondere Bestandteil der erfindungsgemäßen Steuereinrichtung sein. Diese Steuereinrichtung (100) kann insbesondere eingerichtet, insbesondere programmiert sein, die Luftentfeuchtervorrichtung (200) zu steuern, mit der die Inkubatoratmosphäre in einer Inkubatorkammer des Laborgeräts entfeuchtbar ist.

Die Luftentfeuchtervorrichtung kann insbesondere aufweisen oder gebildet sein durch mindestens eine der folgenden Vorrichtungen:

- Luftzuführungseinrichtung, insbesondere Luftpumpeneinrichtung, zur Zuführung von trockener Luft in die Inkubatorkammer des Laborgeräts;
- Kondensationsentfeuchter bzw. Kondensationstrockner;
- Adsorptionsentfeuchter bzw. Adsorptionstrockner;
- Membranentfeuchter bzw. Membrantrockner.

Kondensationstrockner nutzen das Prinzip der Kondensation, um Feuchtigkeit aus der Luft zu entfernen. Die Luft wird durch ein Kühlregister geleitet, wodurch sie abgekühlt wird. Wenn die Luft abgekühlt wird, kondensiert die Feuchtigkeit und wird in einem Auffangbehälter gesammelt oder direkt abgeführt. Die entfeuchtete Luft wird dann vorzugsweise wieder auf die gewünschte Temperatur erwärmt und in die Inkubatorkammer zurückgeführt.

Adsorptionstrockner verwenden adsorbierende Materialien wie Silicagel oder Zeolith, um Feuchtigkeit aus der Luft zu entfernen. Die feuchte Luft wird durch das adsorbierende Material geleitet, wo die Feuchtigkeit an der Oberfläche des Materials adsorbiert wird. Die entfeuchtete Luft wird dann aus dem Trocknungsmaterial herausgeführt und zurück in die Inkubatorkammer geleitet. Das adsorbierende Material kann periodisch regeneriert werden, entweder durch Erwärmung (Thermalregeneration) oder durch eine Gegenströmung trockener Luft (Druckregeneration), um die adsorbierte Feuchtigkeit zu entfernen und das Material wieder einsatzbereit zu machen.

Membrantrockner verwenden eine halbdurchlässige Membran, um Wasserdampfmoleküle von der Luft zu trennen. Die feuchte Luft wird durch die Membran geleitet, wo Wasserdampf durch die Membran hindurch diffundiert, während die trockene Luft auf der anderen Seite herauskommt. Dieses Verfahren erfordert keinen Energieverbrauch für die Regeneration; die Effizienz des Membrantrockners kann von der Temperatur und dem Druck abhängen.

Vorzugsweise ist die Luftentfeuchtervorrichtung eine Luftzuführungsvorrichtung, und die Luftentfeuchtersteuereinrichtung ist dazu eingerichtet, insbesondere programmiert, die Luftzuführungsvorrichtung, insbesondere eine Luftpumpe, zu steuern, mit der eine Umgebungsluft, insbesondere mittels eines Filter gefilterte Umgebungsluft, in die Inkubatorkammer beförderbar ist. Dazu weist das Laborgerät vorzugsweise diese Luftzuführungseinrichtung auf. Die Kammer weist vorzugsweise eine Eintrittsöffnung für die Zufuhr der von der Luftzuführungseinrichtung beförderten Luft in den Kammerinnenraum auf. Diese Luft weist typischer Weise eine geringere Temperatur auf als die Temperatur im Kammerinnenraum und/oder eine geringere Luftfeuchte auf als die Luftfeuchte im Kammerinnenraum. Durch die Zuführung der Luft aus der Umgebung in den Kammerinnenraum lässt sich deshalb die Luftfeuchte und/oder die Temperatur im Kammerinnenraum ändern, insbesondere reduzieren.

Vorzugsweise ist die Luftentfeuchtersteuereinrichtung dazu eingerichtet, insbesondere programmiert, eine Luftentfeuchtervorrichtung, insbesondere eine Luftzuführungseinrichtung, insbesondere eine Luftpumpe, so zu steuern, dass der Luftfeuchtewert im Kammerinnenraum der Inkubatorkammer gesenkt wird.

Vorzugsweise ist die Luftentfeuchtersteuereinrichtung dazu eingerichtet, insbesondere programmiert, eine Luftentfeuchtervorrichtung, insbesondere eine Luftzuführungseinrichtung, insbesondere eine Luftpumpe, so zu steuern, dass ein Luftfeuchtewert rH im Kammerinnenraum der Inkubatorkammer ausgehend ausgehend von einem Messwert rH_start der Luftfeuchte und von einem gemessenen Temperaturwert T_start im Kammerinnenraum in Abhängigkeit von einem Temperaturzielwert Tziel eingestellt wird, wobei der Temperaturzielwert der von einem Benutzer oder ein elektronisch vorgegebener Temperatursollwert ist, auf den die Temperatur im Kammerinnenraum von einer Temperaturregelungseinrichtung der Steuereinrichtung herabgeregelt werden soll. Insbesondere ist der einzustellende Luftfeuchtewert der absoluten Luftfeuchte rH_ziel niedriger als der aktuell vom Luftfeuchtesensor gemessene Luftfeuchtewert absoluter Luftfeuchte rH_start. Der Wert der relativen Luftfeuchte, insbesondere der Luftfeuchtewert x_rH, bleibt in der Kammer vorzugsweise unverändert, vergleicht man den Wert vor Temperaturänderung mit dem Wert nach Temperaturänderung.

Vorzugsweise ist die Luftentfeuchtersteuereinrichtung dazu eingerichtet, insbesondere programmiert, eine Luftentfeuchtervorrichtung, insbesondere eine Luftzuführungseinrichtung, insbesondere eine Luftpumpe, so zu steuern, dass ein Luftfeuchtewert im Kammerinnenraum der Inkubatorkammer in Abhängigkeit von einem Temperaturzielwert Tziel eingestellt wird, wobei der Temperaturzielwert der von einem Benutzer oder ein elektronisch (z.B. Durch Programmierung) vorgegebener Temperatursollwert ist, auf den die Temperatur im Kammerinnenraum von einer Temperaturregelungseinrichtung der Steuereinrichtung geregelt werden soll. Insbesondere ist der einzustellende Luftfeuchtewert der absoluten Luftfeuchte (synonym: absolute Luftfeuchtigkeit) niedriger als der aktuell vom Luftfeuchtesensor gemessene Luftfeuchtewert absoluter Luftfeuchte, insbesondere im Falle einer Temperatursenkung. Der Wert der relativen Luftfeuchte, insbesondere der Luftfeuchtewert x_rH, bleibt in der Kammer vorzugsweise unverändert, vergleicht man den Wert vor Temperatursenkung mit dem Wert nach Temperatursenkung.

Insbesondere wenn der vom Benutzer gewählte Temperaturzielwert T_ziel unter dem aktuellen, vom Temperatursensor der Kammer des Laborgeräts gemessenen Temperaturwert T_start liegt, wird von der Luftentfeuchtersteuereinrichtung die absolute Luftfeuchte gesenkt, insbesondere bevor oder während die Temperatur im Kammerinnenraum auf den Temperaturzielwert gesenkt wird, insbesondere bevor (bzw. ohne dass) von einer Luftfeuchteregeleinrichtung mittels eines Luftfeuchtesensors, der die relative Luftfeuchte im Kammerinnenraum misst, detektiert wird, dass die relative Luftfeuchte den Luftfeuchtesollwert bzw. den damit verbundenen Schwellwert x' überschritten hat. Die durch Temperatursenkung erzeugte Erhöhung der relativen Luftfeuchte wird auf diese Weise insbesondere antizipiert, wenn eine Änderung der relativen Luftfeuchte bei Temperatursenkung nicht erwünscht ist. Die Luftentfeuchtersteuereinrichtung ist dann vorzugsweise dazu eingerichtet, insbesondere programmiert, die Luftentfeuchtervorrichtung, insbesondere die Luftzuführungseinrichtung, so zu steuern, dass der gemessene Luftfeuchtewert x_rH auf einen Luftfeuchtezielwert w'_rH < x_rH gesenkt wird, insbesondere indem die Luftentfeuchtervorrichtung so gesteuert wird, dass einmalig oder kontinuierlich oder sukzessive Luft in dem Kammerinnenraum befördert wird, so dass bzw. bis die Luftfeuchte x_rH den Luftfeuchtezielwert w'_rH oder einen Schwellwert für die Luftfeuchte erreicht, der insbesondere höher liegt, oder tiefer liegt, als der Luftfeuchtezielwert w'_rH. Es findet also vorzugsweise eine Entfeuchtung der Luft während des Temperaturabkühlvorgangs statt (oder unmittelbar davor oder danach).

Die oben beschriebenen Maßnahmen sind für eine effiziente Luftfeuchteeinstellung geeignet. Sie sind insbesondere geeignet, eine unerwünschte Kondensation von Wasser im Kammerinnenraum zu verhindern, welche eintritt, wenn bei einem Absenken der Temperatur (z.B. benutzergesteuert) die mit einer bestimmten (dampfförmigen) Wassermenge angereicherte Inkubatoratmosphäre gekühlt wird und dadurch die relative Luftfeuchte in der Inkubatoratmosphäre erhöht wird. In einer Inkubationskammer mit hoher Luftfeuchte führt eine Temperatursenkung zur Kondensation, weil kältere Luft weniger Feuchtigkeit halten kann als wärmere Luft, und die relative Luftfeuchtigkeit das Verhältnis zwischen dem tatsächlichen Feuchtigkeitsgehalt der Luft und dem maximalen Feuchtigkeitsgehalt bei einer bestimmten Temperatur darstellt. Der Sättigungsdampfdruck ist der Druck, den ein Gas über einer Flüssigkeit ausübt, wenn sich das Gas und die Flüssigkeit im thermodynamischen Gleichgewicht befinden. In Bezug auf Feuchtigkeit ist der Sättigungsdampfdruck der Druck, bei dem die Luft mit Wasserdampf gesättigt ist und kein weiteres Wasser verdunsten kann. Der Sättigungsdampfdruck steigt mit zunehmender Temperatur. Wenn die Temperatur in einer Inkubationskammer gesenkt wird, nimmt der Sättigungsdampfdruck ab, da kältere Luft weniger Wasser aufnehmen kann als wärmere Luft. Wenn der Sättigungsdampfdruck den tatsächlichen Druck in der Kammer übersteigt, kondensiert überschüssige Feuchtigkeit aus der Luft und bildet Wassertropfen oder Feuchtigkeit an Oberflächen. Die relative Luftfeuchtigkeit (rH) steigt ebenfalls, da die Menge an Wasserdampf in der Luft konstant bleibt, während die maximale Menge, die die Luft halten kann, aufgrund der Temperatursenkung abnimmt. Das Verhältnis zwischen dem aktuellen Feuchtigkeitsgehalt und dem maximalen Feuchtigkeitsgehalt bei der neuen Temperatur erhöht sich, was zu einer höheren relativen Luftfeuchtigkeit führt.

Durch die intelligente Einstellung der Luftfeuchte wird insbesondere, wenn bei T_start=37°C bereits auf rH_start 85 % (relative Luftfeuchte) eingeregelt ist und der Nutzer bspw. Tziel = 32 °C und rH_ziel=85 % einstellt, intern ein höherer (virtueller Luftfeuchteoberwert) rH Wert für die Regelung verwendet (hierbei erfolgt eine Umrechnung in absolute Feuchte und dann Berechnung der relativen Feuchte bei 32 °C). In diesem Fall würde vorzugsweise auch die Luftentfeuchtervorrichtung (z.B. Luftpumpe) aktiviert werden bis der intern umgerechnete rH Wert unterhalb der maximal zu tolerierenden rH Schwelle des Gerätes liegt. Es findet also eine Entfeuchtung der Luft während des Temperaturabkühlvorgangs statt

Die Steuereinrichtung oder die Luftentfeuchtersteuereinrichtung ist, gemäß einer Variante A, vorzugsweise dazu programmiert,

a) über eine Benutzerschnittstelle der Steuereinrichtung bzw. der Luftentfeuchtersteuereinrichtung einen vom Benutzer eingegebenen (Benutzertemperatur) Tziel zu erfassen oder einen elektronisch gespeicherten oder vorgegebenen Temperaturzielwert Tziel für den Kammerinnenraum zu verwenden,

b) die aktuell im Kammerinnenraum vorliegende Temperatur T_start zu erfassen, insbesondere über einen Temperatursensor im Kammerinnenraum oder an der Kammerwand anliegend, die aktuell im Kammerinnenraum vorliegende aktuelle Luftfeuchte rH_start zu erfassen, insbesondere über einen Luftfeuchtesensor im Kammerinnenraum,

c) optional einen vom Benutzer eingegebenen Luftfeuchtesollwert (Benutzerluftfeuchte) rH_ziel für den Kammerinnenraum zu erfassen,

d) einen erhöhten Luftfeuchtewert (Luftfeuchteoberwert, relative Luftfeuchte) zu berechnen, der sich einstellt, wenn bei Vorliegen des Luftfeuchtesollwertes oder der aktuellen Luftfeuchte oder der Benutzerluftfeuchte die Benutzertemperatur eingestellt wird,

e) den Luftfeuchteoberwert als virtuellen Messwert zu verwenden, um die Luftfeuchte, insbesondere durch Regelung, auf den Luftfeuchtesollwert rH_ziel zu senken, insbesondere auf die Benutzerluftfeuchte.

Die genannten Luftfeuchtewerte sind jeweils die relative Luftfeuchte im Kammerinnenraum. Im Schritt d) kann die relative Luftfeuchte in eine absolute Luftfeuchte umgerechnet werden, um dann den Luftfeuchteoberwert als relative Luftfeuchte bei der Benutzertemperatur zu messen.

Im Resultat wird die relative Luftfeuchte, wenn im Kammerinnenraum durch separate Temperaturregelung die Benutzertemperatur eingestellt ist, durch die separate Luftfeuchteregelung die gewünschte Luftfeuchte (Luftfeuchtesollwert) vorliegen, ohne dass in der Realität der Luftfeuchteoberwert bzw. irgendein oberhalb des Luftfeuchtesollwerts liegender Luftfeuchtewert im Kammerinnenraum vorliegen wird. Während der Schritte a) bis e) ist die Luftfeuchteregeleinrichtung, mit der eine Verdampfervorrichtung gesteuert wird, inaktiv gestellt. Denn in einer Übergangsphase wird die aktuelle relative Luftfeuchte eventuell auf einen Wert unterhalb der Schwellwerte x und y sinken, welche normalerweise den zweiten oder ersten Betriebsmodus zum Heraufregeln der Luftfeuchte aktivieren würden.

Die oben genannten Schritte d) und e) werden insbesondere einmalig durchgeführt oder wiederholt, bis ausgehend vom virtuellen Messwert, der höher ist als der tatsächliche Messwert der Luftfeuchte, der Luftfeuchtesollwert (z.B. Benutzerluftfeuchte) erreicht ist.

Falls in Verbindung mit den Schritten a) bis e) die Temperatur im Kammerinnenraum nicht auf den niedrigeren Temperaturzielwert gesenkt würde, dann würde die relative Luftfeuchte durch die Schritte a) bis e) im Kammerinnenraum erhöht werden. Da aber die Temperatur gesenkt wird, sinkt zwar die absolute Luftfeuchte (der absolute dampfförmige Wassergehalt) im Kammerinnenraum, die relative Luftfeuchte ist aber (nach erfolgreicher Durchführung der geregelten Temperatursenkung und der un/geregelten Luftfeuchtesenkung) unverändert bzw. entspricht dem Luftfeuchtesollwert.

Die Steuereinrichtung oder die Luftentfeuchtersteuereinrichtung ist, gemäß einer Variante B, vorzugsweise dazu programmiert,

i) über eine Benutzerschnittstelle der Steuereinrichtung bzw. der Luftentfeuchtersteuereinrichtung einen vom Benutzer eingegebenen Temperaturzielwert (Benutzertemperatur) Tziel für den Kammerinnenraum zu erfassen oder einen elektronisch gespeicherten oder vorgegebenen Temperaturzielwert Tziel für den Kammerinnenraum zu verwenden,,

ii) die aktuell im Kammerinnenraum vorliegende Temperatur T_start zu erfassen, insbesondere über einen Temperatursensor im Kammerinnenraum oder an der Kammerwand anliegend, die aktuell im Kammerinnenraum vorliegende aktuelle Luftfeuchte rH_start zu erfassen, insbesondere über einen Luftfeuchtesensor im Kammerinnenraum,

iii) optional einen vom Benutzer eingegebenen Luftfeuchtesollwert (Benutzerluftfeuchte) rH_ziel für den Kammerinnenraum zu erfassen,

iv) die Luftfeuchte (Luftfeuchteunterwert, absolute Luftfeuchte) zu berechnen, die im Kammerinnenraum vorliegen müsste, damit ausgehend von der aktuellen, (absoluten) Luftfeuchte im Kammerinnenraum, eine Temperatursenkung auf den Temperaturzielwert (z.B. Benutzertemperatur) die relative Luftfeuchte auf den Luftfeuchtesollwert erhöht.

v) den Luftfeuchteunterwert als virtuellen Luftfeuchtesollwert zu verwenden, um die Luftfeuchte durch Regelung auf den Luftfeuchtesollwert zu senken, insbesondere auf die Benutzerluftfeuchte.

Im Fall Variante A) erfolgt insbesondere die Luftfeuchtesenkung mit dem virtuellen Messwert der Luftfeuchte als Regelgröße.

Im Fall B) erfolgt die insbesondere Luftfeuchtesenkung mit dem tatsächlichen Messwert der Luftfeuchte als Regelgröße.

In beiden Fällen A) und B) wird die absolute Luftfeuchte auf das für die Temperatursenkung erforderliche Maß gesenkt, wenn eine durch Temperatursenkung verursachte Änderung der relativen Luftfeuchte unerwünscht ist.

Die relative Luftfeuchte (RH) kann mit der folgenden Formel berechnet werden, wenn die Temperatur in einem Raumvolumen von einer Starttemperatur $T$start auf eine Zieltemperatur Tziel gesenkt wird:

$$\mathrm{RH}_{\text{ziel}} = \frac{e_{\text{ziel}}}{e_{\text{sättigung,ziel}}} \times 100\%$$

wobei:

- $e_{\text{ziel}}$ der Wasserdampfdruck bei der Zieltemperatur $T_{\text{ziel}}$ ist.
- $e_{\text{sättigung,ziel}}$ der Sättigungswasserdampfdruck bei der Zieltemperatur $T_{\text{ziel}}$ ist.

Der Sättigungswasserdampfdruck e_sättigung kann durch verschiedene empirische Formeln oder Tabellenwerte in Abhängigkeit von der Temperatur berechnet werden. Eine häufig verwendete empirische Formel ist die Magnus-Tetens-Formel:

$$e_{\text{sättigung}}(T) = 6.1078 \times 10^{(7.5 \times T/(237.3+T))}$$

wobei $T$ die Temperatur in Grad Celsius ist.

Der Wasserdampfdruck e kann durch Messungen oder durch Berechnung unter Verwendung der Clausius-Clapeyron-Gleichung oder anderen empirischen Formeln ermittelt werden, die den Zusammenhang zwischen Temperatur und Feuchtigkeit beschreiben.

Die relative Luftfeuchte gibt das Verhältnis des tatsächlichen Wasserdampfdrucks zum Sättigungswasserdampfdruck bei einer bestimmten Temperatur an und wird üblicherweise in Prozent ausgedrückt.

Die Erfindung betrifft auch ein System, umfassend die Verdampfervorrichtung, welche die Wasserfördereinrichtung und das Heizelement beinhaltet, und eine erfindungsgemäße Steuereinrichtung, und insbesondere den Luftfeuchtesensor zur Messung der Luftfeuchte der Inkubatoratmosphäre in der Kammer des Inkubators und insbesondere den Temperatursensor zur Messung einer Temperatur des Heizelements.

Die Erfindung betrifft auch ein Laborgerät mit Inkubationsfunktion, zur Inkubation lebender Zellkulturen, das eine Verdampfervorrichtung aufweist, welche die Wasserfördereinrichtung und das Heizelement beinhaltet, und das eine erfindungsgemäße Steuereinrichtung aufweist, und das insbesondere den Luftfeuchtesensor zur Messung der Luftfeuchte der Inkubatoratmosphäre in der Kammer des Inkubators und insbesondere den Temperatursensor zur Messung einer Temperatur des Heizelements aufweist.

Die Erfindung betrifft auch Verfahren zur elektronischen Steuerung der Befeuchtung der Inkubatoratmosphäre eines Laborgeräts mit Inkubationsfunktion zur Inkubation lebender Zellkulturen, insbesondere zur Regelung der Luftfeuchte der Inkubatoratmosphäre, mit den Schritten:

- Regeln eines mittels Luftfeuchtesensor messbaren Luftfeuchtewertes der Inkubatoratmosphäre auf einen Luftfeuchtesollwert mittels einer Luftfeuchteregeleinrichtung;

- Regeln einer mittels Temperatursensor messbaren Temperatur eines Heizelements der Verdampfervorrichtung auf eine konstante Zieltemperatur mittels einer Heizregeleinrichtung,
  wobei die Zieltemperatur als eine Verdampfungstemperatur wählbar ist, die geeignet ist, ein mit dem Heizelement kontaktiertes Wasservolumen zu verdampfen;

- Verwenden eines Wasserförderleistungsparameters zur Ansteuerung einer Wasserfördervorrichtung mittels einer Wasserfördersteuereinrichtung, wobei der Wasserförderleistungsparameter ein aktuell zu förderndes Wasservolumen bestimmt,

- Variieren des Wasserförderleistungsparameter in Abhängigkeit von dem Verdampfungswertes, bis ein mittels Luftfeuchtesensor messbarer Luftfeuchtewert der Inkubatoratmosphäre einem Luftfeuchtesollwert entspricht.

Vorzugsweise beinhaltet das Verfahren auch die Schritte:

- Regeln eines mittels Luftfeuchtesensor messbaren Luftfeuchtewertes der Inkubatoratmosphäre auf einen vom Inkubator bzw. dem Benutzer vorgegebenen Luftfeuchtesollwert mittels einer Luftfeuchteregeleinrichtung, wobei als Stellgröße ein Verdampfungssollwert verwendet wird,
  wobei insbesondere dieser Verdampfungssollwert einem Wasservolumen entspricht, insbesondere zu einem Wasservolumen proportional ist, dessen Verdampfung zur Einstellung der Luftfeuchte der Inkubatoratmosphäre erforderlich ist,

- Regeln eines Verdampfungswertes auf den Verdampfungssollwert mittels einer Wasserförderregeleinrichtung der Verdampfervorrichtung, wobei als Stellgröße ein Wasserförderleistungsparameter einer Wasserfördervorrichtung verwendet wird,
  wobei insbesondere dieser Verdampfungswert zu einem aktuell verdampften Wasservolumen korreliert ist, oder diesem entspricht, insbesondere zu diesem proportional ist, und dieser Wasserförderleistungsparameter einem aktuell geförderten Wasservolumen entspricht, insbesondere zu diesem proportional ist,

- Regeln einer mittels Temperatursensor messbaren Temperatur eines Heizelements der Verdampfervorrichtung auf eine konstante Zieltemperatur mittels einer Heizregeleinrichtung, wobei als Stellgröße der Verdampfungswert verwendet wird,
  wobei insbesondere die Zieltemperatur als Verdampfungstemperatur gewählt ist, die geeignet ist, ein während eines Verdampfungsvorgangs mit dem Heizelement in Kontakt kommendes Wasservolumen zu verdampfen und dabei dem Heizelement Wärme zu entziehen, und wobei insbesondere der Verdampfungswert zu einem Wasservolumen proportional ist, das vom Heizelement verdampft wird, und der sich aus einer Messung einer bei dieser Regelung auftretenden Heizleistung oder einer Temperaturabweichung der gemessenen Temperatur von der Zieltemperatur ergibt.

Die Erfindung betrifft auch Verfahren zur elektronischen Steuerung der Befeuchtung der Inkubatoratmosphäre eines Laborgeräts mit Inkubationsfunktion zur Inkubation lebender Zellkulturen dient, insbesondere zur Regelung der Luftfeuchte der Inkubatoratmosphäre, mit den Schritten:

- Regeln eines mittels Luftfeuchtesensor messbaren Luftfeuchtewertes der Inkubatoratmosphäre auf einen vom Inkubator bzw. dem Benutzer vorgegebenen Luftfeuchtesollwert mittels einer Luftfeuchteregeleinrichtung, wobei als Stellgröße ein Verdampfungssollwert verwendet wird,
  wobei insbesondere dieser Verdampfungssollwert zu einem Wasservolumen korreliert bzw. proportional ist, dessen Verdampfung zur Einstellung der Luftfeuchte der Inkubatoratmosphäre erforderlich ist,

- Regeln eines Verdampfungswertes auf den Verdampfungssollwert mittels einer Wasserförderregeleinrichtung der Verdampfervorrichtung, wobei als Stellgröße ein Wasserförderleistungsparameter einer Wasserfördervorrichtung verwendet wird,

wobei insbesondere dieser Verdampfungswert zu einem aktuell verdampften Wasservolumen proportional ist und dieser Wasserförderleistungsparameter zu einem aktuell geförderten Wasservolumen proportional ist,

- Regeln einer mittels Temperatursensor messbaren Temperatur eines Heizelements der Verdampfervorrichtung auf eine konstante Zieltemperatur mittels einer Heizregeleinrichtung, wobei als Stellgröße der Verdampfungswert verwendet wird,

wobei insbesondere die Zieltemperatur geeignet ist, ein während eines Verdampfungsvorgangs mit dem Heizelement in Kontakt kommendes Wasservolumen zu verdampfen und dabei dem Heizelement Wärme zu entziehen, und wobei insbesondere der Verdampfungswert zu einem Wasservolumen proportional ist, das vom Heizelement verdampft wird, und der sich aus einer Messung einer bei dieser Regelung auftretenden Heizleistung oder einer Temperaturabweichung der gemessenen

Temperatur von der Zieltemperatur ergibt.

Die Wasserfördereinrichtung wird vorzugsweise durch eine Pumpeneinrichtung gebildet. Die Pumpeneinrichtung kann eine Mikrodosierpumpe sein, eine Membranpumpe, eine Peristaltikpumpe, eine Schneckenpumpe, oder eine Piezo-Pumpe. Die Dosiereinrichtung kann auch für eine Wasserförderung durch Schwerkraft eingerichtet sein. Dazu weist die Dosiereinrichtung eine Drosseleinrichtung einen entlang der Gravitationsrichtung anordenbaren Wasserzufluss auf, wobei das Wasser insbesondere von einem oberhalb der Dosiereinrichtung anordenbaren Wasserreservoir oder Wasseranschluss stammen kann. Die Drosseleinrichtung ist elektrisch steuerbar, um einen Wasserleitungsquerschnitt und damit den Fluss des Wassers zu steuern.

Vorzugsweise ist die Pumpeneinrichtung zur Erzeugung eines Pumpenhubs eingerichtet, insbesondere derart, dass -im Wesentlichen- jeder Pumpenhub in einem verdampfbaren Tropfen resultiert, der durch die Wassereintrittsöffnung auf die Wasserführungseinrichtung befördert wird und zur Heizfläche gelangt. Damit ist eine kontrollierbare Wasserverdampfung gewährleistet, die in inkrementellen Dosisvolumina erfolgt, entsprechend einem Tropfen oder einer als Wasserfilm abgegeben Wassermenge, die zur Heizfläche gelangt.

Vorzugsweise ist die Dosiereinrichtung durch mindestens eine der folgenden technischen Daten gekennzeichnet:

a) das Dosiervolumen beträgt zwischen 1 $\mu$l und 50 $\mu$l, vorzugsweise zwischen 5 $\mu$l und 20 $\mu$l, vorzugsweise zwischen 10 $\mu$l und 20 $\mu$l, vorzugsweise zwischen 7 $\mu$l und 15 $\mu$l, vorzugsweise zwischen 9 $\mu$l und 11 $\mu$l;
b) die minimale Dosierrate beträgt zwischen 0.5 $\mu$l/h und 100 $\mu$l/h, vorzugsweise zwischen 0.5 $\mu$l/h und 20 $\mu$l/h, vorzugsweise zwischen 0.5 $\mu$l/h und 10 $\mu$l/h.

Da der Verdampfungswert das verdampfte Wasservolumen quantifiziert, ist die Luftfeuchte effizient einstellbar, auch wenn das genaue zu verdampfende Wasservolumen nicht präzise definierbar ist, was z.B. im Falle einer Mikromembranpumpe bei den hier vorgesehenen, kleinsten transportierten Wasservolumina (5 $\mu$l bis 40 $\mu$l) aufgrund von Benetzungseffekten der Fall ist. Es wird somit Flexibilität gewonnen bezüglich der Ausführung der Wasserfördereinrichtung. Es ist aber nicht ausgeschlossen, eine als präzise Dosiereinrichtung ausgeführte Wasserfördereinrichtung zu verwenden und technisch sicherzustellen, dass das geförderte kleinste Wasservolumen auch tatsächlich beim Heizelement ankommt und von diesem vollständig in Dampf umgewandelt wird. Eine solche Dosiereinrichtung könnte insbesondere mittels einer oder mehrerer Kolben/Kolbenkammern realisiert werden.

Vorzugsweise weist die Verdampfervorrichtung eine elektronische Steuereinrichtung auf. Diese ist insbesondere dazu eingerichtet, die Dosiereinrichtung und die Heizfläche zu steuern. Diese Steuereinrichtung ist vorzugsweise dazu eingerichtet, die Dampfabgaberate der Verdampfervorrichtung zu steuern. Diese Steuerung erfolgt mit dem Ziel der Regelung der der Luftfeuchte in einer mit der Dampfaustrittsöffnung verbindbaren Inkubationskammer, insbesondere indem die Dosierleistung der Dosiereinrichtung frequenzgesteuert durch ein elektrisches Signal mit einer Eingangsfrequenz definiert wird.

Die Steuereinrichtung ist insbesondere mit einem Temperatursensor verbunden, der angeordnet ist, um die Temperatur der Heizfläche zu erfassen.

Die Steuereinrichtung ist vorzugsweise dazu eingerichtet, die Temperatur der Heizfläche auf eine Zieltemperatur einzustellen bzw. zu regeln, die insbesondere aus dem Bereich zwischen 100 °C bis 250 °C ausgewählt ist und vorzugsweise zwischen 140 °C und 200°C, vorzugsweise zwischen 160 °C und 185°C beträgt.

Die elektrische Steuereinrichtung weist insbesondere eine Datenverarbeitungseinrichtung auf und ist vorzugsweise dazu programmiert, eine Temperatur einer Heizeinheit, insbesondere der Heizfläche, zu erfassen und insbesondere in Abhängigkeit von dieser Temperatur die Leistung der Heizeinheit einzustellen. Vorzugsweise ist die Steuereinrichtung dazu programmiert, eine Temperatur der Heizeinheit, insbesondere eines Heizblocks oder insbesondere der Heizfläche auf eine gewünschte, insbesondere konstante, Zieltemperatur zu regeln. Vorzugsweise ist die Steuereinrichtung dazu programmiert, einen Heizregelkreis zu bilden, der dazu eingerichtet ist, die mittels eines Temperatursensors gemessene Temperatur eines Heizelements des Verdampfers auf eine konstante Zieltemperatur zu regeln, bei der ein mit dem Heizelement in Kontakt kommendes Wasservolumen verdampft wird und dabei dem Heizelement Wärme entzieht.

Die elektronische Steuereinrichtung ist vorzugsweise dazu programmiert, mindestens eine Funktion der Verdampfervorrichtung und/oder der Luftentfeuchtervorrichtung zu steuern, insbesondere die Zuführung von Wasser durch die Wassereintrittsöffnung mittels der Dosiereinrichtung, und/oder das Heizen der Heizfläche, und/oder das Messen einer Temperatur des Heizelementes. Die elektronische Steuereinrichtung ist vorzugsweise dazu programmiert, Daten zu erfassen, die Informationen eines Sensors des Laborgeräts beinhalten, und insbesondere in Abhängigkeit von diesen Daten die mindestens eine Funktion der Verdampfervorrichtung zu steuern. Der Sensor kann insbesondere ein Luftfeuchtesensor sein, der die Luftfeuchte in der Inkubationskammer des Laborgeräts misst. Der Sensor kann auch ein Türsensor sein, der erfasst, ob eine die Inkubationskammer verschließende Türe geöffnet oder geschlossen ist. Insbesondere kann vorgesehen sein, die Dampfabgaberate der Verdampfervorrichtung in Abhängigkeit von diesen Daten zu ändern, beispielsweise um nach einer Öffnung der Türe (bei dann wieder geschlossener Türe) die Dampfabgaberate schnell zu erhöhen, um die gewünschte relative Luftfeuchte, z.B. 95%, in der Inkubationskammer möglichst schnell wiederherzustellen.

Die Funktionen der Steuereinrichtung sind insbesondere durch Programmcode und/oder durch elektronische Schaltkreise implementiert. Die Steuereinrichtung kann einen Mikrocontroller, eine Recheneinheit (CPU) zum Verarbeiten von Daten bzw. einen Mikroprozessor aufweisen, die jeweils der Datenverarbeitungseinrichtung zugeordnet sein können.

Die Steuereinrichtung kann als selbständig arbeitendes Bauteil ausgebildet sein, dass die Funktionen der Verdampfervorrichtung und/oder der Luftentfeuchtervorrichtung steuert, das aber insbesondere nicht eine oder mehrere Funktionen des Laborgeräts steuert, mit dem die Verdampfervorrichtung verbunden ist oder dessen Bestandteil die Verdampfervorrichtung vorzugsweise ist.

Die Steuereinrichtung kann aber auch durch eine solche Steuereinrichtung gebildet sein, die außer den Funktionen der Verdampfervorrichtung und/oder der Luftentfeuchtervorrichtung auch mindestens eine, mehrere oder alle Funktionen des Laborgeräts steuert, mit dem die Verdampfervorrichtung und/oder die Luftentfeuchtervorrichtung verbunden ist oder dessen Bestandteil die Verdampfervorrichtung und/oder die Luftentfeuchtervorrichtung vorzugsweise ist. Eine der Funktionen des Laborgeräts ist insbesondere die Regelung der Temperatur in der Inkubationskammer des Laborgeräts, oder die Regelung der Gaszusammensetzung in der Inkubationskammer, insbesondere der $CO_2$-Konzentration. Eine der Funktionen des Laborgeräts ist insbesondere auch die Steuerung eines Benutzerschnittstellenmoduls des Laborgeräts, das dem Benutzer Informationen anzeigt, insbesondere über Sensorwerte zu in/an der Inkubationskammer gemessenen physikalischen oder chemischen Größen.

Die Erfindung betrifft ferner ein Laborgerät zur Inkubation von Proben in einer Inkubationskammer, insbesondere einen Inkubator zur Inkubation lebender Zellkulturen oder einen Shaker mit Inkubationsfunktion, das eine erfindungsgemäße Verdampfervorrichtung aufweist.

Der Inkubator ist ein Laborgerät bzw. ein Laborinkubator. Ein Inkubator bezeichnet insbesondere ein Laborgerät mit einer Inkubatorkammer (Inkubationskammer), deren Atmosphäre vom Inkubator auf eine vorgegebene Zieltemperatur regelbar ist bzw. geregelt wird. Insbesondere handelt es sich um ein Laborgerät, mit dem kontrollierte Klimabedingungen für verschiedene biologische Entwicklungs- und Wachstumsprozesse geschaffen und erhalten werden können. Der Inkubator kann ein Schüttelinkubator (Shaker), also ein Inkubator mit einer Bewegungseinrichtung zur Bewegung von in der Inkubatorkammer angeordneten Objekten, sein oder diesen beinhalten. , u Der Inkubator kann insbesondere als eine Zellkultivierungsvorrichtung ausgebildet sein. Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des $CO_2$ und/oder des $O_2$ und/oder des $N_2$-Gehalts des Gases und/oder eine Einstelleinrichtung zur Einstellung der Luftfeuchtigkeit in der Inkubatorkammer des Inkubators.

Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur in der Inkubatorkammer geregelt werden.

Vorzugsweise weist der Inkubator eine Verdampfervorrichtung und/oder eine Luftentfeuchtervorrichtung auf, mittels der die Luftfeuchtigkeit in der Atmosphäre der Inkubatorkammer eingestellt wird.

CO2-Inkubatoren dienen insbesondere der Kultivierung tierischer bzw. humaner Zellen.

Inkubatoren können Wendevorrichtungen zum Wenden des mindestens einen Zellkulturbehälters und/oder eine Schütteleinrichtung zum Schütteln bzw. Bewegen des mindestens einen Zellkulturbehälters aufweisen. Der erfindungsgemäße Inkubator ist insbesondere nicht ein Bioreaktor oder Fermenter.

Der Inkubator kann mindestens eine Sensoreinrichtung aufweisen. Eine Sensoreinrichtung weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an $CO_2$ und/oder $O_2$ und/oder $N_2$. Eine Sensoreinrichtung weist vorzugsweise einen Sensor zur Ermittlung der relativen Luftfeuchtigkeit auf.

Ein Inkubator weist vorzugsweise eine bzw. eine einzige Inkubatorkammer auf. Diese kann in Kompartimente unterteilt sein. Kompartimente können durch -insbesondere gelochte-Lagerplatten getrennt sein, wobei insbesondere ein Gasaustausch zwischen den Kompartimenten ermöglicht ist.

Die Inkubatorkammer weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Diese Kammeröffnung ist durch ein mit der Inkubatorkammer beweglich verbundenes Verschlusselement verschließbar, insbesondere eine mittels Schwenktüre (eine zum Öffnen vollständig von der Kammeröffnung nach oben weggleitende Türe, "slide-up Mechanismus) oder mittels Scharnier an der Inkubatorkammer beweglich montierte Inkubatortüre, insbesondere eine oder mehrere Kammertüren. Ein Inkubator kann eine oder mehrere Innentüren aufweisen, die insbesondere transparent sein können, und kann eine -insbesondere nicht transparente- Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert.

In der verschlossenen Position der Kammeröffnung ist das Innere der Inkubatorkammer gegen die Umgebung vorzugsweise in der Weise isoliert, so dass im Inneren eine gewünschte, vom Inkubator gesteuerte Temperatur bzw. Atmosphäre einstellbar, insbesondere regelbar ist. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand des Inkubators.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, Kupfer, Messing, oder ein Kuns-stoff, insbesondere ein Verbundkunststoff. Dadurch wird die Reinigung/ Desinfektion des Kammerinneren erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Kabelverbindung aus dem Inneren der Inkubatorkammer an deren Außenseite oder in die Umgebung des Inkubators aufweisen.

Eine Seitenwand der Inkubatorkammer weist insbesondere eine Dampfeintrittsöffnung auf, die mit der Dampfaustrittsöffnung der Verdampfervorrichtung verbunden ist, insbesondere fluiddicht verbunden ist. Diese Seitenwand der Inkubatorkammer weist insbesondere auch einen Befestigungsabschnitt zur Befestigung der Verdampfervorrichtung auf. Der Befestigungsabschnitt kann die Dampfeintrittsöffnung beinhalten, in der vorzugsweise ein Verbindungselement zur Verbindung der Verdampfervorrichtung mit der Seitenwand angeordnet ist.

Eine typische Größe des Inneren einer Inkubatorkammer liegt zwischen 50 und 400 Litern (dm3).

Der Inkubator kann genau eine Inkubatorkammer aufweisen, kann aber auch mehrere Inkubatorkammern aufweisen, deren Luftfeuchte der Atmosphäre (oder: Temperatur, relative Gaskonzentration) insbesondere individuell oder gesammelt einstellbar sein kann. Ein Inkubator kann mehrere Inkubatorkammern aufweisen, die jeweils eine eigene Kammeröffnung und eine eigene Kammertüre zum Verschließen der Kammeröffnung aufweisen können. Jede dieser Inkubatorkammern, oder eine Gruppe dieser Inkubatorkammern, kann mit einer Verdampfervorrichtung verbunden sein, um die Luftfeuchte individuell oder gruppenweise einzustellen bzw. zu regeln.

Der Inkubator kann ein Gehäuse aufweisen, das die Inkubatorkammer teilweise oder vollständig umgibt. Eine erfindungsgemäße Verdampfervorrichtung ist vorzugsweise, insbesondere neben der Inkubatorkammer, innerhalb des Gehäuses angeordnet. Das Gehäuse kann im Wesentlichen quaderförmig ausgebildet sein, und kann insbesondere derart ausgebildet sein, dass der Inkubator stapelbar ist.

Die Erfindung betrifft auch ein System aus einer Anzahl N>1 an Inkubatoren, und mindestens einer erfindungsgemäßen Verdampfervorrichtung, insbesondere einer Anzahl M>=1 von erfindungsgemäßen Verdampfervorrichtungen, vorzugsweise M=N, die jeweils individuell mit einer Inkubatorkammer verbunden sind. Vorzugsweise weist das System ein Wasserreservoir auf, das insbesondere oberhalb oder unterhalb eines Stapels der N Inkubatoren angeordnet ist. Das Wasserreservoir ist mit der Dosiereinrichtung jeder der Verdampfervorrichtungen verbunden. Das System ist insbesondere so eingerichtet, dass im Betrieb mehrerer dieser Inkubatoren Wasser aus diesem einen Wasserreservoir von den Verdampfervorrichtungen dieser Inkubatoren verdampft wird.

Weitere bevorzugte Ausgestaltungen der erfindungsgemäßen Gegenstände, insbesondere des erfindungsgemäßen Verfahrens, lassen sich aus der Beschreibung der erfindungsgemäßen Steuereinrichtung bzw. des Laborgeräts mit Inkubationsfunktion und deren bevorzugten Ausgestaltungen entnehmen. Ferner ergeben sich weitere Ausgestaltungsoptionen der Erfindung aus den Ausführungsbeispielen in den Figuren. Gleiche Teile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:

Fig. 1 zeigt einen Querschnitt durch eine perspektivische Seitenansicht der Verdampfervorrichtung gemäß einem Ausführungsbeispiel der Erfindung, in montierter Position an der Seitenwand einer Inkubationskammer eines Inkubators, ohne die Dosiereinrichtung.

Fig. 2a zeigt in schematischer Frontansicht ein Ausführungsbeispiel einer von der erfindungsgemäßen Steuereinrichtung gesteuerten Verdampfervorrichtung an einem Laborgerät gemäß einem Ausführungsbeispiel der Erfindung.

Fig. 2b zeigt in schematischer Frontansicht ein System aus einem Stapel von Laborgeräten mit der durch die erfindungsgemäßen Steuereinrichtung gesteuerten Verdampfervorrichtungen gemäß Fig. 2a, die mit einem gemeinsamen Wasserreservoir verbunden sind.

Fig. 2c zeigt ein Ausführungsbeispiel eines mit einer erfindungsgemäßen Steuereinrichtung versehenen Laborgeräts, das ein Inkubationsschüttler ist, an dessen Inkubatorkammerwand die Verdampfervorrichtung der Fig. 1a montiert ist.

Fig. 2d zeigt in schematischer Frontansicht ein Ausführungsbeispiel einer von einer Steuereinrichtung gesteuerten Luftentfeuchtervorrichtung an einem Laborgerät gemäß einem Ausführungsbeispiel einer weiteren Erfindung.

Fig. 3 zeigt schematisch die Bestandteile einer erfindungsgemäßen Steuereinrichtung gemäß einem Ausführungsbeispiel.

Fig. 4 zeigt schematisch Komponenten einer von einer beispielhaften, erfindungsgemäßen Steuereinrichtung angesteuerten Verdampfervorrichtung zur Regelung der Luftfeuchte rH in der Inkubatorkammer eines Laborgeräts mit Inkubationsfunktion.

Fig. 5 zeigt ein Schaubild, in dem die Funktionsweise einer beispielhaften, erfindungsgemäßen Steuereinrichtung im Falle des ersten Betriebsmodus dargestellt ist, bei dem die Wasserfördereinrichtung kontinuierlich angesteuert wird und ein kontinuierlicher Dampfstrom produziert wird.

Fig. 6a zeigt den zeitlichen Verlauf der gemessenen Luftfeuchte $x\_rH$ und der dabei aufgebrachten Pumpleistung $y\_WP$ über einen Zeitraum, ausgehend von der Situation, das die Inkubatorkammer längere Zeit geöffnet war und dann bei geschlossener Kammertüre die Luftfeuchteregelung gemäß dem ersten Betriebsmodus der beispielhaften, erfindungsgemäßen Steuereinrichtung durchgeführt wurde.

Fig. 6b zeigt den zeitlichen Verlauf der im Verlauf der Luftfeuchteregelung gemäß Fig. 6a gemessenen Temperatur des Heizelements, der Heizleistung $y\_TC$ des Heizelements und des Verdampfungssollwerts $w\_WP$.

Fig. 7 zeigt ein Schaubild, in dem die Funktionsweise der Steuereinrichtung im Falle des zweiten Betriebsmodus dargestellt ist, bei dem die Wasserfördereinrichtung diskontinuierlich angesteuert wird und ein diskontinuierlicher Dampfausstoß produziert wird.

Fig. 8a zeigt den zeitlichen Verlauf der gemessenen Luftfeuchte $x\_rH$ und der dabei aufgebrachten Pumpleistung $y\_WP$ über einen Zeitraum, ausgehend von der Situation, das die Inkubatorkammer längere Zeit geschlossen war und dann bei geschlossener Kammertüre die Luftfeuchteregelung gemäß dem zweiten Betriebsmodus der beispielhaften, erfindungsgemäßen Steuereinrichtung durchgeführt wurde.

Fig. 8b zeigt den zeitlichen Verlauf der im Verlauf der Luftfeuchteregelung gemäß Fig. 8a gemessenen Temperatur des Heizelements und der Heizleistung $y\_TC$ des Heizelements.

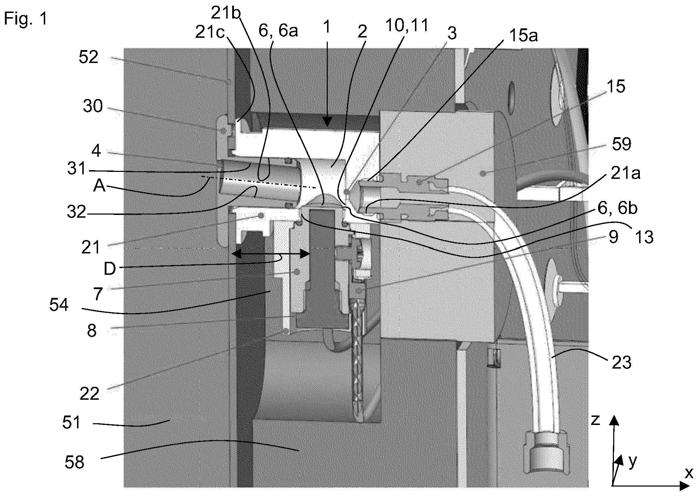

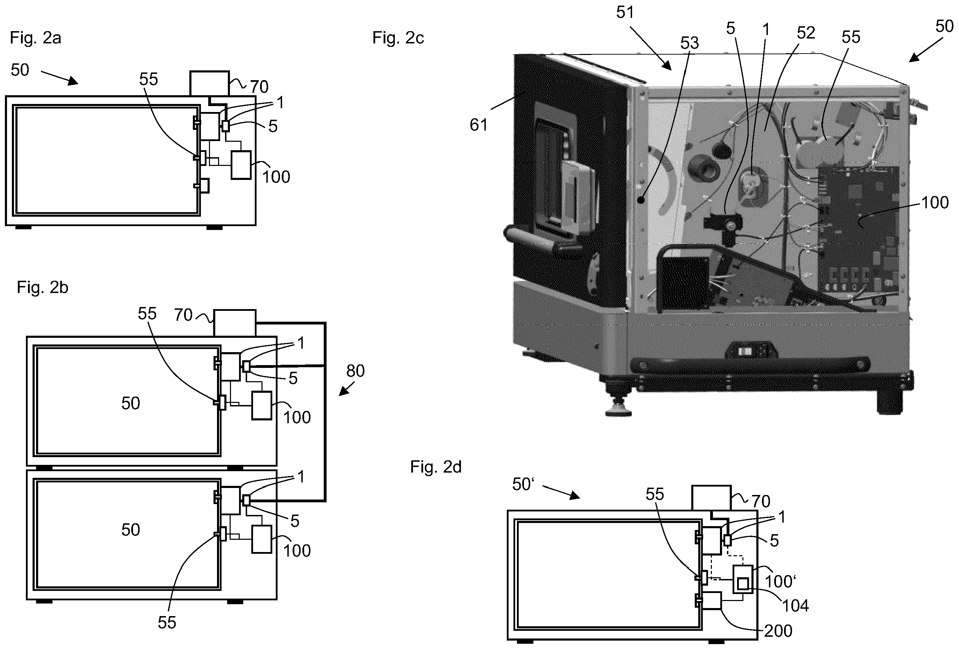

Fig. 1 zeigt eine von einer erfindungsgemäßen Steuereinrichtung steuerbare Verdampfervorrichtung 1, in montierter Position an der Seitenwand 52 einer Inkubationskammer 51 eines COz-Inkubators 50 zur Inkubation von Zellkulturen, der in Fig. 2c gezeigt ist. Die negative z-Richtung entspricht der Gravitationsrichtung. Die Verdampfervorrichtung 1 dient der Befeuchtung der Inkubatoratmosphäre in der Inkubationskammer. Die Verdampfervorrichtung 1 weist eine Verdampferkammer 2 auf, deren Bodenabschnitt eine Heizfläche 6 enthält, die mit dem Innenraum der Verdampferkammer in Kontakt ist und die somit in der Verdampferkammer 2 angeordnet ist und mit der ein mit der Heizfläche in Kontakt kommendes Wasser verdampfbar ist. Die Verdampfervorrichtung 1 weist ein erstes Bauteil 21 auf. Dieses enthält die hier durch Drehen aus einem gefrästen Bauelement gefertigte Verdampferkammer 2, welche eine Wassereintrittsöffnung 3 zur Zuführung von flüssigem Wasser in die Verdampferkammer und eine mit dem Innenraum einer Inkubatorkammer des Inkubators verbindbare Dampfaustrittsöffnung 4 aufweist.

Die Wasserfördereinrichtung 5, hier eine Mikromembranpumpe, mit der Wasser zu der Wassereintrittsöffnung beförderbar ist, ist nicht gezeigt. Die Dosiereinrichtung 5 wird über die Wasserleitung 23 an das Wasseranschlusselement 15 angeschlossen. Das Wasseranschlusselement 15 weist eine zylinderförmigen Mündungsabschnitt 15a auf, der in einem zylinderförmigen Aufnahmeraum 21a des ersten Bauteils 21 aufgenommen ist, der in die Wassereintrittsöffnung 3 mündet; sowohl der Mündungsabschnitt 15a als auch der Aufnahmeraum 21a münden somit in der Wassereintrittsöffnung 3.

Die Verdampfervorrichtung 1 weist eine Wasserführungseinrichtung 10 mit einer Wasserführungsfläche 11 auf, die durch einen Innenwandabschnitt 11 der Verdampferkammer gebildet wird. Mittels der Wasserführungseinrichtung 10 wird das durch die Wassereintrittsöffnung 3 in die Verdampferkammer 2 eingetretene Wasser bis zu der Heizfläche 6 an der Wasserführungseinrichtung 10 entlang fließend geführt.

Im unteren, zweiten Bauteil 22, das mit dem ersten Bauteil 21 verbunden ist, befindet sich die Heizpatrone 8, die in dem Heizelement 7 bzw. Heizblock 7 eingehaust ist. Zur Temperaturregelung ist ein Temperatursensor 9 an dem Heizblock 7 befestigt. Das Wasser gelangt über die kleine Drosselbohrung 3 in die Verdampferkammer 2. Anschließend läuft der eingeleitete Tropfen an der Wandung 11 herunter auf den beheizten Heizblock und wird dort verdampft.

Die Wasserfördereinrichtung, hier die Pumpe, kann so gewählt und eingerichtet sein, dass im Wesentlichen jeder Pumpenhub in einem verdampfbaren Tropfen resultiert, um den relativen Luftfeuchtigkeitswert (auch: rH-Wert) möglichst fein und gleichmäßig einregeln zu können. Würden sich erst große Tropfen bilden, bevor es zu einem Ablösen und Verdampfen kommt, kann es durch das explosionsartige Verdampfen dieses Tropfens zum Herausschleudern von kleineren Tropfen führen. Zudem ist die Gefahr größer, dass es zu einem Überschießen des rH-Werts in der Kammer kommt. Vorliegend ist die Pumpe mit derart geringer Förderleistung betreibbar, dass mehrere Pumpenhübe ausgeführt werden, um ein erwünschtes kleines Wasservolumen von z.B. 2-15 Mikroliter zu fördern. In den Vorversuchen der Erfinder hatte sich zudem gezeigt, dass es vorteilhaft ist, wenn der Tropfen ohne Fall zur Heizfläche gelangt, indem er insbesondere unmittelbar an der Wand 11 herunterläuft. Die Oberflächenspannung des Wassers wird in dieser Anordnung früher überwunden, so dass es zu einem Ablaufen des Wassers in Richtung des Heizblocks 7 kommt.

In Fig. 1 auf der linken Seite befindet sich die Dampfaustrittsöffnung 4 in die Inkubatorkammer 51. Die Dampfaustrittsöffnung 4 befindet sich oberhalb der Längsachse A des Dampfauslasskanals 31, der mittels Verbindungselement 30 an der Kammerwand 52 befestigt ist. Kondensierendes Wasser in der Dampfaustrittsöffnung 4 soll über die schräge Bodenwand 32 zurück zum Heizblock gefördert werden. Zwischen Inkubatorkammer 51 und Heizpatrone 8 ist ein Abstand D vorgesehen, um einen Übergriff der Temperatur auf die Kammerwand 52 zu reduzieren. Zum weiteren Wärmeschutz kann in den Hohlraum 54 Isoliermaterial eingelegt werden, zudem können dort parallel zur Seitenwand ausgerichtete Plattenelemente 25 des Bauteils 21/21' zur thermischen Abschirmung zwischen dem Heizblock 7 und der Seitenwand 52 vorgesehen werden. Zudem sind beide Bauteile (Gehäuseteile) 21, 22 aus einem schlecht wärmeleitenden Material (hier Kunststoff PEEK) gefertigt. Um die Energieeffizienz zu erhöhen sowie zur besseren Entkopplung von Umgebungseinflüssen ist der Verdampfer nach außen hin durch Silikonisolierschaumteile 58, 59 isoliert. Die Isolierung 58, 59 kann zu Reparaturzwecken einfach entnommen werden.

Fig. 2a zeigt einen Inkubator 50 mit Verdampfervorrichtung 1. Das Wasser der Wasserpumpe 5 wird aus einem Wasserreservoir 70 bezogen, das oberhalb der Wasserförder- bzw. Pumpeneinrichtung 5, hier auf der Gehäuseoberseite des Inkubators 50 angeordnet ist.

Fig. 2b zeigt ein System 80 aus einem Stapel von Inkubatoren 50, deren Verdampfervorrichtungen mit einem gemeinsamen Wasserreservoir 70 verbunden sind. Das Wasserreservoir 70 ist oberhalb jeder Pumpeneinrichtung 5, hier auf der Gehäuseoberseite des obersten Inkubators 50 angeordnet. Das Wasserreservoir 70 ist mit der Pumpeinrichtung 5 jeder der Verdampfervorrichtungen 1 verbunden. Das System 80 ist insbesondere so eingerichtet, dass im Betrieb mehrerer dieser Inkubatoren Wasser aus diesem einen Wasserreservoir 70 von allen Verdampfervorrichtungen 1 dieser Inkubatoren verdampft wird.

Fig. 2c zeigt das als Inkubator 50 bzw. Inkubationsschüttler 50 ausgebildete Laborgerät 50, an dessen Inkubatorkammerwand 52 die Verdampfervorrichtung 1 der Fig. 1 montiert ist. Die Inkubatorkammer 51 ist durch die Türe 61 verschlossen. Diese weist einen Türsensor 53 auf, mit der der Öffnungsstatus der Türe ermittelbar ist. Das Gehäuse des Laborgeräts ist größtenteils nicht gezeigt. Ein Vorteil der Erfindung ist, dass die Tankposition des Wasserreservoirs relativ zum Laborgerät in gewissen Grenzen frei wählbar ist, da die Steuereinrichtung insbesondere einen höheren hydrostatischen Druck (Gerät im Stapel ganz unten, Tank ganz oben) "erkennt" und die Förderleistung entsprechend anpasst.

Fig. 2d zeigt ein von einer Steuereinrichtung 100' gesteuerte Luftentfeuchtervorrichtung 200 an einem Laborgerät 50' gemäß einem Ausführungsbeispiel einer weiteren Erfindung. Das Wasser der Wasserpumpe 5 wird auch hier aus einem Wasserreservoir 70 bezogen, das oberhalb der Wasserförder- bzw. Pumpeneinrichtung 5, hier auf der Gehäuseoberseite des Inkubators 50' angeordnet ist. Die Steuereinrichtung 100' kann, wie hier der Fall, auch dazu eingerichtet sein, die Verdampfervorrichtung 1 zu steuern - die Optionalität dieser Maßnahme ist durch die gestrichelten Linien angezeigt. Andererseits kann auch vorgesehen sein, dass die Steuereinrichtung zur elektronischen Einstellung der Luftfeuchte, insbesondere der Luftfeuchte der Inkubatoratmosphäre des Inkubators 50' zur Inkubation lebender Zellkulturen mittels einer Verdampfervorrichtung 1, in die Verdampfervorrichtung 1 integriert ist (nicht gezeigt). Die Steuereinrichtung 100' kann alle Bestandteile und die gesamte Funktionalität der Steuereinrichtung 100 aufweisen, und zusätzlich die Luftentfeuchtersteuereinrichtung 104. Die Steuereinrichtung 100' kann insbesondere eine Luftfeuchteregeleinrichtung (101), eine Heizregeleinrichtung (102), und eine Wasserfördersteuereinrichtung (103) aufweisen, wie dies gemäß einer erfindungsgemäßen Ausführungsform der Steuereinrichtung 100 definiert ist. Die Funktionsweise der Luftentfeuchtersteuereinrichtung wurde bereits vorher beschrieben.

Falls mittels des Luftfeuchtesensors 55 eine Temperatur gemessen wird, die über einem zulässigen Schwellwert oberhalb des Luftfeuchtesollwerts liegt, ist die Steuereinrichtung 100', insbesondere die Luftentfeuchtersteuereinrichtung 104 der Steuereinrichtung 100', vorzugsweise dazu eingerichtet und programmiert, eine Luftentfeuchtervorrichtung 200 so zu steuern, dass die Luftfeuchte in der Kammer 2 des Laborgeräts gesenkt wird, insbesondere bis der genannte Schwellwert (x') wieder unterschritten wird oder der Luftfeuchtesollwert erreicht wird. Dies erfolgt insbesondere durch Regelung der Luftfeuchte, wobei als Stellglied der Regelung diese Luftentfeuchtervorrichtung 250 dient. Diese kann eine Luftzuführeinrichtung, insbesondere eine Luftpumpeneinrichtung, aufweisen, mit der Luft in die Kammer befördert wird, die eine geringere Luftfeuchte aufweist als in der Kammer gemessen wurde. Der genannte Schwellwert x' kann insbesondere in einem Bereich von vorzugsweise 0,01-1,0%, vorzugsweise 0,01-0,4 %, vorzugsweise 0,5 - 0,3%, vorzugsweise 0,1 - 0,25%, vorzugsweise 0,15 - 0,25% (gemeint ist jeweils Prozent relative Luftfeuchte) über dem Luftfeuchtesollwert liegen, also zum Beispiel als Schwellwert x': 95,2% relative Luftfeuchte bei einem Luftfeuchtesollwert von 95% relative Luftfeuchte mit x'=0,2%. Die Verdampfervorrichtung und/oder die Luftentfeuchtervorrichtung, hier eine Kolbenpumpe, werden vorzugsweise auch so betrieben, dass ein durch Temperaturänderung bewirkte Änderung der relativen Luftfeuchte vermieden bzw. in gewünschter Weise auf einen Zielwert adaptiert wird. Dies erfolgt insbesondere mittels geeigneter Programmierung der Luftentfeuchtersteuereinrichtung 104 zur Ansteuerung der Luftentfeuchtervorrichtung 200. Diese Funktionsweise der Luftentfeuchtersteuereinrichtung wurde bereits vorher beschrieben.

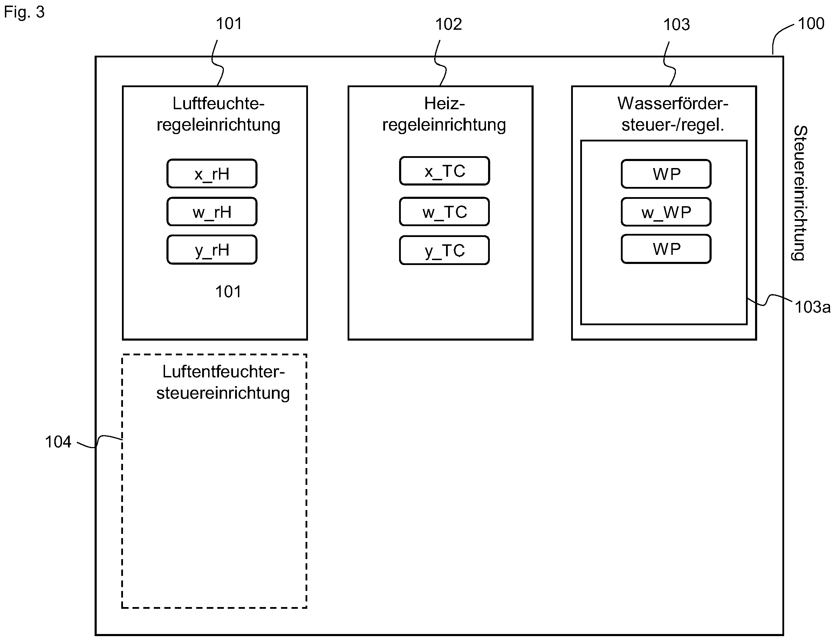

Fig. 3 zeigt schematisch die Bestandteile einer erfindungsgemäßen Steuereinrichtung 100 gemäß einem Ausführungsbeispiel, die der elektronischen Einstellung der Luftfeuchte einer Inkubatoratmosphäre in der Inkubationskammer 51 eines Inkubators 50 zur Inkubation lebender Zellkulturen mittels einer Verdampfervorrichtung 1 dient.

Die Steuereinrichtung 100 weist eine Luftfeuchteregeleinrichtung 101 auf, die dazu eingerichtet, insbesondere programmiert ist, den Luftfeuchtewert $x\_rH$ (Regelgröße) auf einen Luftfeuchtesollwert $w\_rH$ (Sollwert) zu regeln.

Die Steuereinrichtung 100 weist eine Heizregeleinrichtung 102 auf, die dazu eingerichtet, insbesondere programmiert ist, die mittels eines Temperatursensors 9 messbare Temperatur $x\_TC$ (Regelgröße) eines wasserverdampfenden Heizelements 7 der Verdampfervorrichtung 1 auf eine konstante Zieltemperatur $w\_TC$ (Sollwert) zu regeln, und dabei als Stellgröße $y\_TC$ einen Verdampfungswert zu verwenden.

Der Verdampfungswert wird durch ein Wasservolumen bestimmt, das vom Heizelement verdampft wird. Denn der Stellwert, der nötig ist, um das Heizelement auf z.B. der Zieltemperatur von 180 °C zu halten, ist hier direkt proportional zu der Leistung, die in Wärme umgewandelt wird. Die nötige Leistung wiederum ist hier direkt proportional zu der Menge an Wasser, die im Mittel dem Verdampfer zugeführt wird. Der Verdampfungswert kann deshalb auch als Wasservolumen-Verdampfungs-Leistungswert angesehen und bezeichnet werden.

Der Verdampfungswert ergibt sich hier aus einer Messung einer bei dieser Regelung auftretenden Heizleistung. Diese ist insbesondere proportional zum Tastverhältnis einer Pulsweitenmodulation (PWM), mit dem das Heizelement betrieben wird.

Alternativ zu der Heizleistung kann auch eine Temperaturabweichung der gemessenen Temperatur von der Zieltemperatur zur Bestimmung des Verdampfungswertes verwendet werden. Im letztgenannten Fall kann eine Konkordanzliste angelegt und in einer Datenspeichereinrichtung der Steuereinrichtung gespeichert sein, die jeder Temperaturabweichung einen Verdampfungswert zuordnet.

Als Zieltemperatur wird zum Zwecke des Verdampfens eine Verdampfungstemperatur gewählt, die geeignet ist, ein mit dem Heizelement kontaktiertes Wasservolumen zu verdampfen, z.B. 180° C. Das Heizelement kann auch auf andere Zieltemperaturen geregelt werden, z.B. auf eine Absenktemperatur, die zum Zwecke des Energiesparens deutlich geringer als die Verdampfungstemperatur sein kann, z.B. 60 °C.

Die Steuereinrichtung 100 weist eine Wasserfördersteuereinrichtung 103 auf, die dazu eingerichtet, insbesondere programmiert, ist, einen Wasserförderleistungsparameter WP zur Ansteuerung einer Wasserfördervorrichtung zu verwenden, der ein aktuell zu förderndes Wasservolumen bestimmt. Die Wasserfördersteuereinrichtung kann insbesondere eine Wasserförderregeleinrichtung 103a aufweisen. Deren Parameter, insbesondere deren Stellgröße kann, abhängig vom Betriebsmodus der Steuereinrichtung 100, unterschiedlich gewählt sein.

Die Steuereinrichtung 100 ist dazu eingerichtet, insbesondere programmiert, den Wasserförderleistungsparameter WP in Abhängigkeit von dem Verdampfungswert zu variieren, bis ein mittels Luftfeuchtesensor 55 messbarer Luftfeuchtewert der Inkubatoratmosphäre einem Luftfeuchtesollwert entspricht.

Da der Verdampfungswert das verdampfte Wasservolumen quantifiziert, ist die Luftfeuchte effizient einstellbar, auch wenn das genaue zu verdampfende Wasservolumen nicht präzise definierbar ist, was z.B. im Falle einer Mikromembranpumpe bei den hier vorgesehenen, kleinsten transportierten Wasservolumina (5 μl bis 40 μl) aufgrund von Benetzungseffekten der Fall ist. Es wird somit Flexibilität gewonnen bezüglich der Ausführung der Wasserfördereinrichtung. Es ist aber nicht ausgeschlossen, eine als präzise Dosiereinrichtung ausgeführte Wasserfördereinrichtung zu verwenden und technisch sicherzustellen, dass das geförderte kleinste Wasservolumen auch tatsächlich beim Heizelement ankommt und von diesem vollständig in Dampf umgewandelt wird. Eine solche Dosiereinrichtung könnte insbesondere mittels einer oder mehrerer Kolben/Kolbenkammern realisiert werden.

Die Steuereinrichtung kann, muss aber nicht, eine Luftentfeuchtersteuereinrichtung 104 zur Entfeuchtung der Atmosphäre der Kammer des Laborgeräts aufweisen, wie bereits beschrieben.

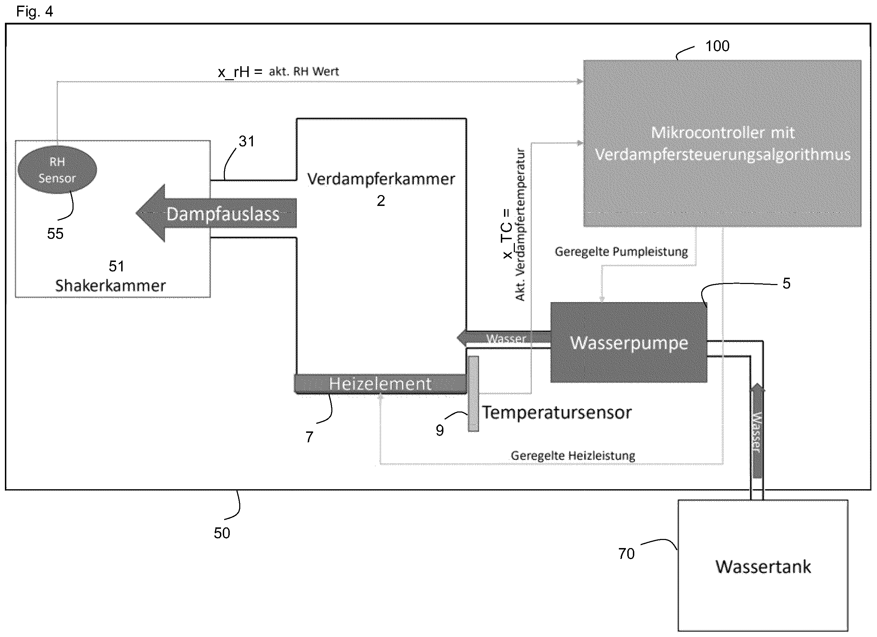

Fig. 4 zeigt schematisch Komponenten einer von einer beispielhaften, erfindungsgemäßen Steuereinrichtung 100 angesteuerten Verdampfervorrichtung zur Regelung der Luftfeuchte rH in der Inkubatorkammer 51 eines Laborgeräts 50 mit Inkubationsfunktion. Die gezeigten Gerätekomponenten wurden bereits anhand der Figuren 1 bis 2c erläutert, die hier als Microcontroller mit Programmcode (Verdampfungssteueralgorithmus) ausgeführte Steuereinrichtung 100 ist anhand Fig. 3 und allen nachfolgenden Figuren genauer erläutert. Die Steuereinrichtung 100 verwendet, wie gezeigt, den Luftfeuchtewert x_rH, nämlich hier den aktuell mittels Luftfeuchtesensor 55 in der Inkubatorkammer 51 gemessenen Wert der Luftfeuchte. Die Steuereinrichtung 100 verwendet zudem, wie gezeigt, die mittels Temperatursensor 9 gemessene Temperatur x_TC des wasser-verdampfenden Heizelements 7. Die Einstellung der Temperatur auf den Temperatursollwert w_TC, hier 180° C, erfolgt geregelt. Die Leistung der Wasserfördereinrichtung 5 erfolgt hier ebenfalls geregelt.

Die Steuereinrichtung 100 ist dazu eingerichtet, insbesondere programmiert, unter anderem

i) in einem ersten Betriebsmodus betrieben zu werden und
ii) in einem zweiten Betriebsmodus betrieben zu werden.

Der erste Betriebsmodus dient insbesondere dazu, nach einer Türöffnung der Türe 61 der Inkubationskammer 51 die Luftfeuchtigkeit möglichst schnell und dennoch ohne signifikante Überschwinger, welche zu Kondensation führen könnten, einzuregeln.

Der zweite Betriebsmodus dient insbesondere dazu, kleinste Wassermengen im geschlossenen Zustand der Türe 61 und im zuvor eingeregelten Zustand der Luftfeuchte in der Inkubationskammer 51 nachzuführen, um die konstante (geringe) Leckage der Feuchtigkeit aus der Inkubationskammer im geschlossenen Zustand der Türe 61 auszugleichen.

Im ersten Betriebsmodus erfolgt die Einstellung der Luftfeuchte durch eine Luftfeuchteregelung und die Einstellung des Wasserförderleistungsparameters durch eine Wasserförderregelung.

Im zweiten Betriebsmodus erfolgt die Einstellung der Luftfeuchte ebenfalls durch eine Luftfeuchteregelung, aber die Einstellung des Wasserförderleistungsparameters WP erfolgt nicht reguliert, wie noch erläutert wird.

Im ersten Betriebsmodus wird die Wasserfördereinrichtung jedenfalls zeitweise und/oder überwiegend, kontinuierlich betrieben. Im zweiten Betriebsmodus wird die Wasserfördereinrichtung diskontinuierlich betrieben, hier gemäß einem intermittierenden Betrieb bzw. Pulsbetrieb der Wasserfördereinrichtung, bei dessen Betriebspulsen jeweils nur kleine Volumeninkremente gefördert werden, wie noch erläutert wird.

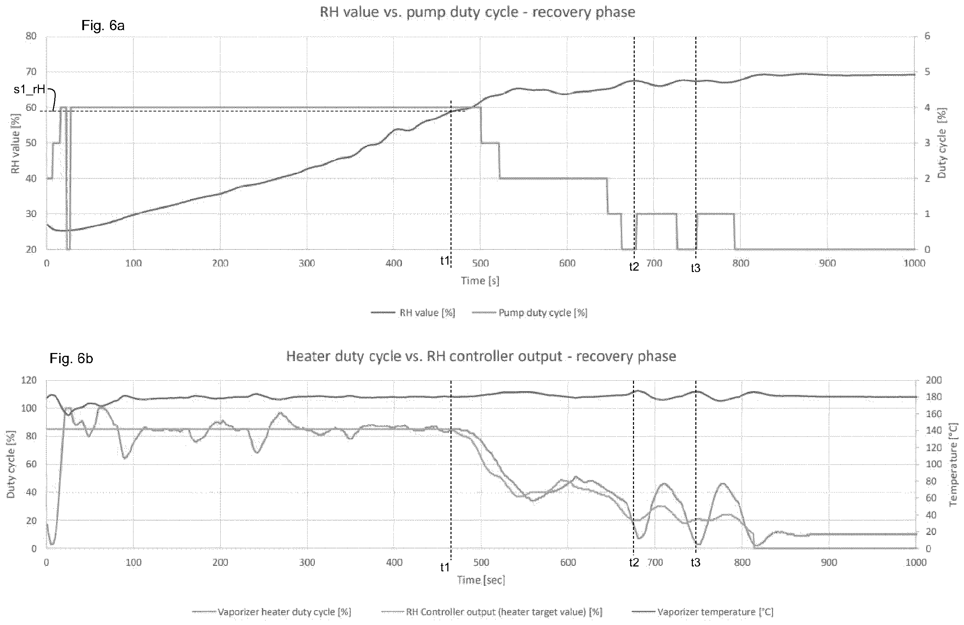

Der kontinuierliche Betrieb der Wasserfördereinrichtung erfordert, dass die Wasserfördereinrichtung für eine Mindestdauer t_min1 ohne Unterbrechung angesteuert wird, während der der Wasserförderleistungsparameter, also z.B. ein Tastverhältnis einer PWM-angesteuerten Pumpe, größer ist als Null. In der Fig. 6a ist zu sehen, dass das Tastverhältnis für ca. t_min1=650s größer ist als Null und die Pumpe demnach in dieser Zeit fortdauernd angesteuert wird - auch wenn die Pumpe während der Zyklen der PWM-Ansteuerung in jeder Periodendauer außerhalb der Pulsdauer abgeschaltet ist.

Die Steuereinrichtung 100 ist insbesondere dazu eingerichtet, insbesondere programmiert, in dem zweiten Betriebsmodus betrieben zu werden, wenn der Luftfeuchtewert x_rH einen ersten Schwellwert s1_rh unterschreitet, und in dem ersten Betriebsmodus betrieben zu werden, wenn der Luftfeuchtewert x_rH einen zweiten Schwellwert s2_rh unterschreitet, wobei der zweite Schwellwert s2_rh niedriger ist als der erste Schwellwert s1_rh.

Im laufenden Betrieb des Laborgeräts 50 mit geschlossener Inkubatorkammertüre 61 und im eingeregelten Zustand der Luftfeuchte kann zum Beispiel eine Nachregelung der Luftfeuchte x_rH dadurch initiiert werden, dass die Luftfeuchte, aufgrund von langsamer Leckage, unter einen ersten, von der Steuereinrichtung 100 beobachteten Schwellwert s1_rh sinkt. Dieser wird in Abhängigkeit von der Empfindlichkeit der in der Kammer angeordneten Proben, insbesondere lebenden Zellen, relativ gering gewählt: insbesondere wird der erste Schwellwert s1_rh in einem Bereich von vorzugsweise 0,01-5 %, vorzugsweise 0,01-1%, unter dem Luftfeuchtesollwert liegen.

Der zweite Betriebsmodus der Steuereinrichtung 100 kann insbesondere dann aktiviert werden, wenn die gemessene Luftfeuchte relativ weit unter den Luftfeuchtesollwert w_rH gesunken ist, insbesondere unter den zweiten Schwellwert s2_rh. Dies kann insbesondere unmittelbar nach einer erfolgten Türöffnung der Fall sein, wenn die Türe 61 wieder geschlossen ist, was von der Steuereinrichtung 100 mittels Türsensor detektierbar ist. Bei geöffneter Türe 61 erfolgt keine Luftfeuchteregelung, das Heizelement wird dann zudem auf eine Absenktemperatur geregelt. Der der zweite Schwellwert s2_rh kann in einem Bereich von vorzugsweise 5-100 % unter dem Luftfeuchtesollwert w_rH liegen.

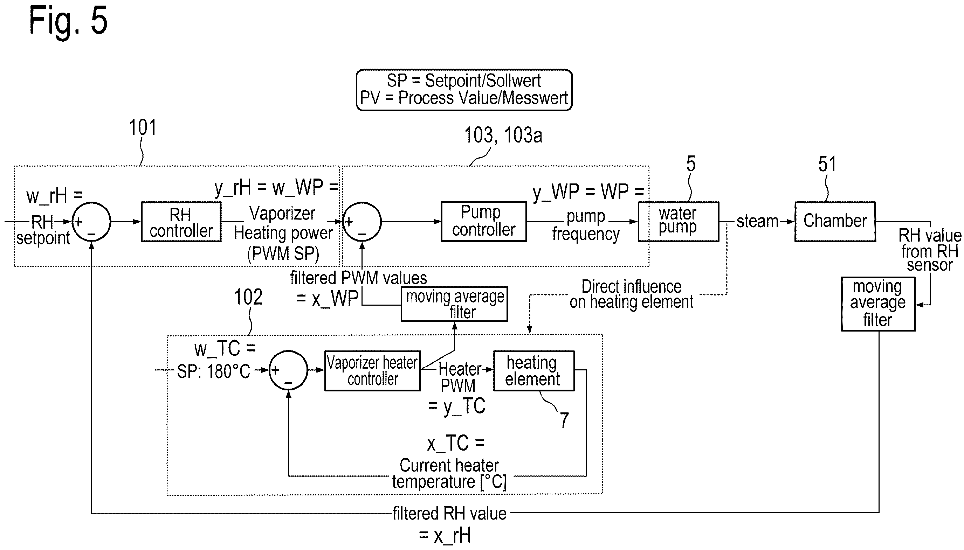

Fig. 5 zeigt ein Schaubild, in dem die Funktionsweise der Steuereinrichtung 100 im Falle des ersten Betriebsmodus dargestellt ist, bei dem die Wasserfördereinrichtung kontinuierlich angesteuert wird und ein kontinuierlicher Dampfstrom produziert wird.

Gemäß der Figur 5 weist die Steuereinrichtung 100 zur elektronischen Steuerung der Befeuchtung der Inkubatoratmosphäre eine Luftfeuchteregeleinrichtung 101 auf, die dazu eingerichtet, insbesondere programmiert, ist, einen mittels Luftfeuchtesensor 55 messbaren Luftfeuchtewert x_rH der Inkubatoratmosphäre auf einen vom Inkubator bzw. dem Benutzer vorgegebenen Luftfeuchtesollwert w_rH (hier: 180° C) zu regeln, und dabei als Stellgröße y_rH einen Verdampfungssollwert w_WP zu verwenden. Der Verdampfungssollwert w_WP wird durch ein Wasservolumen quantifiziert, und ist insbesondere zu diesem proportional, dessen Verdampfung zur Einstellung der Luftfeuchte der Inkubatoratmosphäre erforderlich ist bzw. als von der Steuereinrichtung aufgrund der Abweichung x_rH - w_rH als erforderlich berechnet wird.

Gemäß der Figur 5 weist die Steuereinrichtung 100 eine Wasserförderregeleinrichtung 103a auf, die dazu eingerichtet, insbesondere programmiert, ist, einen Verdampfungswert x_WP auf den Verdampfungssollwert w_WP zu regeln, und dabei als Stellgröße y_WP einen Wasserförderleistungsparameter WP der Wasserfördereinrichtung 5 zu verwenden. Dabei ist dieser Verdampfungswert x_WP zu einem aktuell verdampften Wasservolumen proportional und dieser Wasserförderleistungsparameter WP zu einem aktuell geförderten Wasservolumen proportional. Der Wasserförderleistungsparameter WP kann eine variable Förderfrequenz (z.B. Pumpfrequenz) bzw. Drehzahl sein. Ebenso bevorzugt ist der Wasserförderleistungsparameter WP ein Tastverhältnis (Englisch: "duty cycle") einer mit PWM-modulierter Betriebsspannung betriebener Wasserfördereinrichtung (z.B. Pumpe), die mit konstanter Förderfrequenz ("fixed pump frequency") betrieben wird. Die Frequenz des Tastverhältnisses kann auch verwendet sein, um die Frequenz der Pumpe vorzugeben. Vorzugsweise kommt für diese im Ausführungsbeispiel verwendete Pumpe eine Pulsweitenmodulation (PWM) mit variabler Frequenz zum Einsatz. Dabei wird auch der duty cycle mit der Frequenz verändert, da die Einschaltzeit bei dieser konkreten Pumpe fest vorgegeben ist.

Der Verdampfungswert x_WP wird von der Heizregeleinrichtung geliefert:
Gemäß der Figur 5 weist die Steuereinrichtung 100 eine Heizregeleinrichtung 102 auf, die dazu eingerichtet, insbesondere programmiert, ist, die mittels eines Temperatursensors 9 messbare Temperatur eines Heizelements 7 der Verdampfervorrichtung 1 auf eine konstante Zieltemperatur w_TC (hier: 180° C) zu regeln, und dabei als Stellgröße y_TC den (gemittelten oder nicht gemittelten) Verdampfungswert zu verwenden.

Die Zieltemperatur w_TC ist geeignet, ein bei einem Verdampfungsvorgang mit dem Heizelement 7 in Kontakt kommendes Wasservolumen zu verdampfen und dabei dem Heizelement 7 Wärme zu entziehen. Der Verdampfungswert x_WP ist insbesondere zu einem Wasservolumen proportional, das vom Heizelement 7 verdampft wird. Der Verdampfungswert x_WP ergibt sich hier aus einer Messung einer bei dieser Heizegelung auftretenden Heizleistung. Diese wird vorliegend durch ein Tastverhältnis eines PWM-angesteuerten Heizelements definiert, das mit PWM-modulierter, konstanter Spannung betrieben wird.

Die Arbeitsweise der Steuereinrichtung 100 im kontinuierlichen Betrieb lässt sich kurz so beschreiben: Die Luftfeuchteregeleinrichtung 101 detektiert eine zu geringe Luftfeuchte und fordert deshalb ein Wasser(dampf)volumen (y_rH) an. Die Wasserfördereinrichtung 103 kann das Wasservolumen liefern, kennt aber das genaue Wasservolumen nicht, das zur Verdampfung gelangt, und benötigt deshalb selber diese Information, um die Förderleistung y_WP anzupassen. Diese Information wird vom Heizelementregelkreis 102 geliefert, indem ein Ersatzparameter x_WP ausgegeben wird, der das zur Verdampfung gelangte Wasservolumen bestimmt. Der Ersatzparameter ist die hier die Heizleistung (PWM), die benötigt wird, um die Temperatur konstant zu halten.

Durch das gemeinsame Wirken dreier Regelkreise 101, 102, 103 zugunsten einer kontinuierlichen Wasserförderleistung und Dampfproduktion lässt sich die Luftfeuchte auch nach einem größeren Abfall der gemessenen Luftfeuchte effizient einregeln, insbesondere ohne Überschwingen und somit mit reduziertem Risiko der Kondensatbildung. Das wird in den Figuren 6a und 6b gezeigt.

Fig. 6a zeigt den zeitlichen Verlauf der gemessenen Luftfeuchte x_rH und der dabei aufgebrachten Pumpleistung y_WP ("pump duty cycle", bei PWM-angesteuerter Pumpe; = Wasserförderleistungsparameter WP) über einen Zeitraum, ausgehend von der Situation, das die Inkubatorkammer längere Zeit geöffnet war und dann bei geschlossener Kammertüre die Luftfeuchteregelung gemäß dem ersten Betriebsmodus der Steuereinrichtung 100 durchgeführt wurde.

Fig. 6b zeigt dazu den zeitlichen Verlauf der im Verlauf der Luftfeuchteregelung gemäß Fig. 6a gemessenen Temperatur ("Vaporizer temperature") des Heizelements, der Heizleistung "Vaporizer heater duty cycle" (Verdampfungswert: y_TC = x_WP; "heater PWM" in Fig. 5) des Heizelements und des Verdampfungssollwerts ("RH controller output (heater target value)", y_rH = w_WP, "Vaporizer heating power (PWM SP) in Fig. 5).

Die gemessene Luftfeuchte x_rH ("RH value") startet zum Zeitpunkt 0s bei ca. 27%. Zu diesem Zeitpunkt wird der Verdampfungssollwert y_rH von der Luftfeuchteregeleinrichtung angefordert, der einem bestimmten Wasservolumen entspricht, das wiederum zu einer bestimmten Heizleistung (y_TC = x_WP) des Heizelements äquivalent wäre, wenn die entsprechende Wassermenge dort verdampft würde. Diese Größe wird als Verdampfungswert bezeichnet. Diese angeforderte Verdampfungsleistung lässt sich durch das Tastverhältnis ("PWM SP", siehe Fig. 5) des PWM-angesteuerten Heizelements ausdrücken. In Fig. 6b ist diese Größe mit "RH controller output (heater target value)" bezeichnet und liegt hier bis zum Zeitpunkt ca. t1 = 470s bei konstant ca. 85%, abzulesen an der linken Ordinate (Bezeichnung: "Duty cycle") des Diagramms. Die Temperatur "Vaporizer temperature" liegt zum Zeitpunkt 0s beim Sollwert von 180 °C, abzulesen an der rechten Ordinate im Diagramm. Die Pumpleistung y_WP liegt zum Zeitpunkt 0s bei einem Tastverhältnis von 2%, abzulesen an der rechten Ordinate des Diagramms der Fig. 6a ("Duty cycle").

Durch die große Differenz zwischen Verdampfungswert x_WP und Verdampfungssollwert w_WP zum Zeitpunkt 0s wird die Pumpleistung bis zum Zeitpunkt ca 25s schnell auf einen Maximalwert 4% des Tastverhältnisses angehoben (Fig. 6a, rechte Ordinate "duty cycle"). Alternativ ließe sich auch eine Pumpfrequenz anheben, wie dies durch den Eintrag "pump frequency" in Fig. 5 bei y_WP angedeutet ist. Durch die hohe Pumpleistung schießt auch der Wert für y_TC (Verdampfungswert) nach oben, da das kontinuierlich am Heizelement eintreffende Wasservolumen dem Heizelement Wärme entzieht und das Heizelement in Folge dessen die Heizleistung maximiert. Der Verdampfungswert/die Heizleistung folgt somit unmittelbar dem maximal eingestellten Verdampfungssollwert w_WP nach.

Die Steuereinrichtung 100 ist dazu eingerichtet, insbesondere programmiert, den Luftfeuchtewert x_rH so zu regeln, dass der Luftfeuchtewert dem Luftfeuchtesollwert w_rH =5% (Fig. 6a, rechte Ordinate) asymptotisch angenähert wird, so dass insbesondere ein Überschwingen verhindert wird. Dazu wird vorliegend der Verdampfungssollwert w_WP reduziert, wenn die gemessene Luftfeuchte x_rH einen Schwellwert s1_rH überschreitet, der hier bei ca. 58% liegt, was ca. 83 Prozent des Luftfeuchtesollwertes w_rH = hier 70% entspricht, und hier zum Zeitpunkt ca. t1 = 465s gemessen wird. Der Verdampfungssollwert wird auf Basis des Luftfeuchtewertes geregelt. Das Maximum ist jedoch auf 85 % begrenzt um das System nicht "in Sättigung" zu betreiben, d.h. das auch Abweichungen nach oben (zu viel Leistung) erkannt werden um die Pumpleistung zu reduzieren. Ab t1 fordert die Regelung (bedingt durch P und I Parametrierung) weniger als dieses Maximum an, sodass ein veränderlicher Verlauf sichtbar wird. Die Pumpenleistung scheint zum Zeitpunkt t1 bis t=500s noch nicht reduziert zu werden und noch bis zum Zeitpunkt 500s maximal zu fördern. Diese Darstellung ist dadurch bedingt, dass die Ausgabe des Tastverhältnisses der Pumpe nur auf ganze Prozentzahlen erfolgt, bzw. stufenweise von 5% auf 4%, auf 3%, auf 2%, auf 1 % und auf Null , während intern eine wesentlich feinere Parametrierung des Tastverhältnisses verwendet wird, die hier ("extern") nicht sichtbar wird. Entsprechend wird die Pumpleistung, dem Verdampfungssollwert folgend, herabgeregelt, aber zu den Zeitpunkten ca. t2= 660s und t3= 750s nochmals vorübergehend, einem vorübergehenden Abfall der Luftfeuchte folgend, heraufgesetzt.

Infolge der kontinuierlichen Wasserförderung bis zum Zeitpunkt 500s ergibt sich dort auch ein schnell ansteigender Luftfeuchtewert x_rH (Fig. 6a, linke Ordinate). Ab da nähert sich der Luftfeuchtewert asymptotisch dem Luftfeuchtesollwert (70%), ohne diesen zu übersteigen.

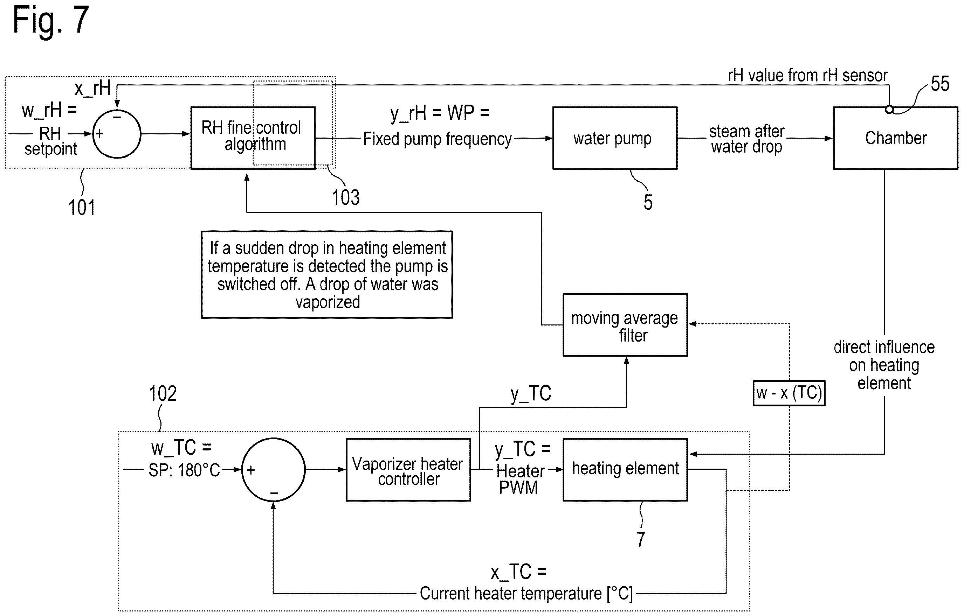

Fig. 7 zeigt ein Schaubild, in dem die Funktionsweise der Steuereinrichtung 100 im Falle des zweiten Betriebsmodus dargestellt ist, bei dem die Wasserfördereinrichtung diskontinuierlich angesteuert wird und ein diskontinuierlicher Dampfausstoß produziert wird.

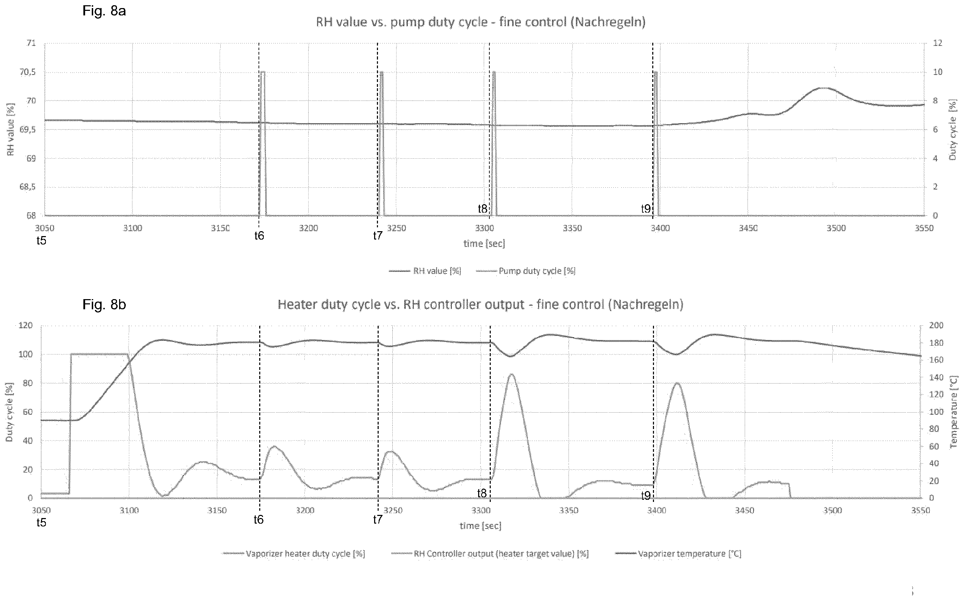

Fig. 8a zeigt den zeitlichen Verlauf der gemessenen Luftfeuchte x_rH ("RH value", blaue Kurve) und der dabei aufgebrachten Pumpleistung y_WP ("pump duty cycle", bei PWM-angesteuerter Pumpe; = Wasserförderleistungsparameter WP, orange Kurve) über einen Zeitraum beginnend zum Zeitpunkt 3050s bis 3550s, also über eine Dauer von ca. 500s, ausgehend von der Situation, das die Inkubatorkammer längere Zeit geschlossen war, dass eine geringe Leckage in der Inkubatorkammer zu einem Abfall der Luftfeuchte von 70% (Sollwert) auf 69,6% geführt hat, einem Schwellwert, dessen Unterschreiten ein Nachregeln der Luftfeuchte im zweiten Betriebsmodus initiiert hat.

Fig. 8b zeigt dazu den zeitlichen Verlauf der im Verlauf der Luftfeuchteregelung gemäß Fig. 8a gemessenen Temperatur ("Vaporizer temperature") des Heizelements und der Heizleistung "Vaporizer heater duty cycle" (Verdampfungswert: y_TC = x_WP; "heater PWM" in Fig. 5) des Heizelements. Im Vergleich zur Regelung in Fig. 6b wird hier der Verdampfungssollwert nicht verwendet ("RH controller output (heater target value)", y_rH = w_WP, "Vaporizer heating power (PWM SP) in Fig. 5).

Zum Zeitpunkt 3050s befindet sich das Heizelement auf der Absenktemperatur von ca. 95 °C. In den Sekunden danach sinkt die Luftfeuchte in der Kammer unter einen Schwellwert von 69,6%, die Steuereinrichtung veranlasst darauf hin zunächst, dass das Heizelement wieder auf Verdampfungstemperatur 180 °C geregelt wird, was durch den plötzlichen Anstieg des "Vaporizer heater duty cycle" (orange Kurve in Fig. 8b) auf 100% erkennbar ist. Dies erfolgt zwischen den Zeitpunkten ca. 3060s und 3115s (siehe Fig. 8b, blaue Kurve "Vaporizer temperature"). Bevor die Pumpe aktiviert wird (vor den Zeitpunkten t6, t7, t8, t9), ermittelt die Steuereinrichtung immer wieder, ob die Heiztemperatur (vaporizer temperature in Fig. 8b) konstant ist, und startet die Pumpe zu diesen Zeiten, weil dann jeweils die Heiztemperatur konstant ist. Die Pumpe wird jeweils so lange mit duty cycle 10% betrieben, bis im Parameter "vaporizer heater duty cycle" (Fig. 8b) ein Anstieg der Heizleistung erkannt wird. Dazu kommt es also nicht auf den höchsten Punkt der Heizleistung an, den die Heizregelung aufgrund des Eintreffens/Verdampfens eines entsprechend der Pumpleistung geförderten Wasservolumens erfordert.

Die Pumpleistung y_rH = WP bildet hier unmittelbar die Stellgröße für die Luftfeuchteregelung. Die Wasserförderung erfolgt hier nicht geregelt, sondern wird durch die

Luftfeuchteregeleinrichtung, welche die Wasserfördersteuereinrichtung bildet, angesteuert und eingestellt.

Diese Art der Ansteuerung der Pumpe zu den Zeiten t6, t7, t8, t9 wird auch als Pulsbetrieb der Wasserfördereinrichtung bezeichnet. Diese Ansteuerung ist speziell auf diese Pumpe abgestimmt und sorgt dafür, dass die Pumpe nur sehr geringe Wasservolumina fördert. Es lässt sich im Vergleich der Fig. 8a und 8b feststellen, dass die zum Zeitpunkt t7 von der Luftfeuchteregeleinrichtung initiierte Wasserabgabe offensichtlich eine wesentlich geringere Wassermenge zum Heizelement führt als die zeitversetzte nächste Wasserabgabe zum Zeitpunkt t8 = 3310s. Die Luftfeuchteregeleinrichtung detektiert die Wasserabgabe zum Zeitpunkt t6 und t7, es wird aber kein Anstieg des Luftfeuchtewertes x_rH ("RH value" in Fig. 8a) detektiert- der entsprechende Wert x_TC wird von der Luftfeuchteregeleinrichtung ausgewertet. Die Luftfeuchteregeleinrichtung ist dazu programmiert, im Falle erfolgloser Tropfenabgabe (wenn der Luftfeuchtewert x_rH nicht ansteigt oder und ein Schwellwert der Luftfeuchte nicht überschritten wurde) einen weiteren Betriebspuls der Pumpe auszuführen, und zwar sobald die Temperatur des Heizelements (vaporizer temperature in Fig. 8b) wieder als konstant angesehen wird: die Wasserabgabe zum Zeitpunkt t6 war erfolglos, die nächste Abgabe zum Zeitpunkt t7 erfolgt ca 120s später. Auch die Wasserabgabe zum Zeitpunkt t7 führte nicht zu einer Erhöhung der Luftfeuchte, die nächste Abgabe zum Zeitpunkt t8 erfolgt wieder ca 120s später und führt wieder nicht zu einer Erhöhung der Luftfeuchte. Der Zeitraum bis t9 ist dann relativ lang, da die Heizelementtemperatur länger benötigt, um wieder konstant zu verlaufen. Dies ist bei t9 der Fall. Da infolgedessen nach dem Pumpenbetrieb bei t9 von der Luftfeuchteregeleinrichtung beobachtet wird, dass die Luftfeuchtigkeit ansteigt und da der Luftfeuchtewert zudem über einen dafür vorgesehenen Schwellwert von hier 69,75% läuft, wird kein weiterer Pumpenbetrieb initiiert. Die Luftfeuchte steigt zwischen 3450s und 3500s auf etwas mehr als den Sollwert w_rH=70% , und läuft dann auf den gewünschten Sollwert 70% zu, um dort konstant zu bleiben, so dass der zweite Betriebsmodus beendet wird. Jetzt wird grundsätzlich das Heizelement wieder auf seine Absenktemperatur geregelt.

Die Wasserförderung erfolgt im zweiten Betriebsmodus nicht automatisch gemäß einem kontinuierlichen Feedback und ist somit ungeregelt. Dagegen ist die Luftfeuchte geregelt, wobei die Stellgröße für die Luftfeuchteregelung unmittelbar der Wasserförderleistungsparameter WP ist, der vom Programm der Luftfeuchteregeleinrichtung festgelegt wird, um damit die Pumpe anzusteuern.

Die Erfindung gemäß bevorzugter Ausführungsform ermöglicht eine robuste und präzise Regelung der Luftfeuchtigkeit in allen Betriebszuständen eines Laborgeräts zur Inkubation, insbesondere eines Inkubationsschüttlers oder Inkubators. Insbesondere das schnelle Einregeln ohne signifikante Überschwinger sowie das Nachführen kleinster Wassermengen im statischen Zustand (z.B. ~40 μL um die Feuchtigkeit von 84,6 % auf 85 % bei 225 l Kammervolumen und 37 °C zu erhöhen) sind hier von Vorteil. Dies wird dadurch erreicht, dass sich innerhalb des Verdampfers 1 nur immer so viel Wasser befindet, wie innerhalb kürzester Zeit verdampft werden kann. Dies wird durch die Einbeziehung des aktuell anliegenden Verdampfungswertes, z.B. der Heizleistung, in die Steuerung bzw. Regelung der Wasserförderleistung der Wasserfördereinrichtung bewerkstelligt. Wird kein Dampf mehr benötigt wird die Wasserfördereinrichtung abgestellt und es verbleibt keine größere Menge Wasser innerhalb des Verdampfers, die zu einem längeren Nachlaufverhalten führen würde. Folglich kommt es nicht zur Kondensatentwicklung in der Inkubatorkammer. Kondensat in der Inkubationskammer stellt eine Kontaminationsgefahr dar, da Mikroorganismen sich darin ansiedeln können. Überdies benötigt diese algorithmengesteuerte Regelung keinen Füllstandsensor in einem Wassertank, da durch eine über einen gewissen Zeitraum zu niedrig angeforderte Heizleistung am Heizelement festgestellt werden kann, dass kein Wasser im Verdampfer ankommt. Ein weiterer Vorteil ist, dass kein Durchflusssensor im Zuführungsschlauch oder am Verdampfer selbst nötig ist, was zu Einsparungen bei den Herstellkosten führt. Dies wird dadurch erreicht, dass die Dampfmenge, die der Verdampfer 1 produziert, dadurch bestimmt wird, wie viel Leistung das Heizelement verbrauchen soll (Verdampfungssollwert). Diese Zielgröße wird insbesondere durch eine Regelung der Wasserfördergeschwindigkeit (Frequenz-/Drehzahlvariation oder Tastverhältnis bei einer PWM-angesteuerten Wasserfördereinrichtung mit konstanter Frequenz) eingehalten. Die algorithmengesteuerte Regelung ist also in der Lage, einen vergleichbaren Betrieb herzustellen, auch wenn z.B. pro Wasserförderzyklus die doppelte Menge Wasser in den Verdampfer gefördert wird, weil durch eine erhöhte Tankposition und die Gravitation ein erhöhter Durchfluss besteht. Durch die beschriebene Funktionalität ist es außerdem möglich einen kontinuierlichen Dampfstrahl unterschiedlicher Intensität zu erzeugen bzw. den maximal zugeführten Dampf pro Zeiteinheit zu begrenzen falls es das System, welches es zu befeuchten gilt, erfordert. Für die beschriebene Erkennung von Wassertropfen ist in dieser Erfindung außerdem nur ein Temperatursensor erforderlich, der gleichzeitig für die Temperaturregelung der Heizfläche verwendet wird. Außerdem sei abschließend noch einmal explizit herausgestellt, dass diese Steuereinrichtung es wahlweise ermöglicht, kontinuierlich Dampf zu erzeugen.

**Patentansprüche**

1. Steuereinrichtung (100) zur elektronischen Einstellung der Luftfeuchte einer Inkubatoratmosphäre eines Laborgeräts (50) zur Inkubation lebender Zellkulturen mittels einer Verdampfervorrichtung (1), aufweisend

   - eine Luftfeuchteregeleinrichtung (101), die dazu eingerichtet ist, den Luftfeuchtewert (x_rH) auf einen Luftfeuchtesollwert (w_rH) zu regeln,
   - eine Heizregeleinrichtung (102), die dazu eingerichtet ist, die mittels eines Temperatursensors (9) messbare Temperatur (T) eines wasserverdampfenden Heizelements (7) der Verdampfervorrichtung (1) auf eine konstante Zieltemperatur (Tsoll) zu regeln, und dabei als Stellgröße einen Verdampfungswert (x_WP; y_TC) zu verwenden,

   und wobei sich insbesondere der Verdampfungswert (x_WP; y_TC) aus einer Messung einer bei dieser Regelung auftretenden Heizleistung oder einer Temperaturabweichung der gemessenen Temperatur von der Zieltemperatur ergibt, wobei insbesondere der Verdampfungswert (x_WP; y_TC) durch ein Wasservolumen bestimmt wird, das vom Heizelement verdampft wird, und
   wobei die Zieltemperatur als Verdampfungstemperatur wählbar ist, die geeignet ist, ein mit dem Heizelement kontaktiertes Wasservolumen zu verdampfen;

   - eine Wasserfördersteuereinrichtung (103), die dazu eingerichtet ist, einen Wasserförderleistungsparameter (y_WP; WP) zur Ansteuerung einer Wasserfördereinrichtung (5) zu verwenden, der ein aktuell zu förderndes Wasservolumen bestimmt,
   - wobei die Steuereinrichtung dazu eingerichtet ist, den Wasserförderleistungsparameter (y_WP; WP) in Abhängigkeit von dem Verdampfungswert (x_WP; y_TC) zu variieren, bis ein mittels Luftfeuchtesensor (55) messbarer Luftfeuchtewert (x_rH) der Inkubatoratmosphäre einem Luftfeuchtesollwert (w_rH) entspricht.

2. Steuereinrichtung gemäß Anspruch 1, die dazu eingerichtet ist,

   in einem ersten Betriebsmodus betrieben zu werden und/oder in einem zweiten Betriebsmodus betrieben zu werden,
   wobei im ersten Betriebsmodus die Einstellung des Wasserförderleistungsparameters durch eine Wasserförderregelung erfolgt,
   wobei insbesondere im zweiten Betriebsmodus die Einstellung des Wasserförderleistungsparameters nicht reguliert erfolgt.

3. Steuereinrichtung gemäß Anspruch 2, die dazu eingerichtet, insbesondere programmiert, ist,

   dass im ersten Betriebsmodus der Verdampfungswert zur Bestimmung der Regelgröße (x_WP) der Wasserförderregelung verwendet wird und insbesondere ein Verdampfungssollwert (w_WP) als Stellgröße der Wasserförderregelung verwendet wird, und insbesondere
   im zweiten Betriebsmodus der Wasserförderleistungsparameter (WP) als Stellgröße (y_rH) der Luftfeuchteregelung verwendet wird, der insbesondere von der Steuereinrichtung in Abhängigkeit von dem Verdampfungswert eingestellt wird.

4. Steuereinrichtung gemäß Anspruch 3,

   - wobei die Luftfeuchteregeleinrichtung dazu eingerichtet, insbesondere programmiert, ist, in einem ersten Betriebsmodus der Steuereinrichtung einen Verdampfungssollwert (x_WP; y_TC) als Stellgröße zu verwenden,

   und insbesondere in einem zweiten Betriebsmodus der Steuereinrichtung den Wasserförderleistungsparameter (WP) als Stellgröße zu verwenden,
   wobei insbesondere dieser Verdampfungssollwert (x_WP; y_TC) ein Wasservolumen bestimmt, dessen Verdampfung zur Einstellung der Luftfeuchte der Inkubatoratmosphäre erforderlich ist, wobei insbesondere der Luftfeuchtesollwert vom Inkubator bzw. dem Benutzer vorgegeben wird;

   - wobei die Wasserfördersteuereinrichtung eine Wasserförderregeleinrichtung aufweist, die dazu eingerichtet ist, in einem ersten Betriebsmodus der Steuereinrichtung den Verdampfungswert (x_WP; y_TC) auf den Verdampfungssollwert (y_rH = w_WP) zu regeln, und dabei als Stellgröße (y_WP) den Wasserförderleistungsparameter (WP) zu verwenden.

5. Steuereinrichtung gemäß einem der Ansprüche 1 bis 4, die dazu eingerichtet, insbesondere programmiert, ist,

in einem ersten Betriebsmodus betrieben zu werden und/oder in einem zweiten Betriebsmodus betrieben zu werden,
und in dem ersten Betriebsmodus die Wasserfördereinrichtung kontinuierlich zu betreiben, und insbesondere in dem zweiten Betriebsmodus die Wasserfördereinrichtung diskontinuierlich, insbesondere in einem Pulsbetrieb, zu betreiben.

6. Steuereinrichtung gemäß einem der Ansprüche 2 bis 5, die dazu eingerichtet ist, in dem ersten Betriebsmodus betrieben zu werden, wenn der Luftfeuchtewert einen ersten Schwellwert unterschreitet, und dem zweiten Betriebsmodus betrieben zu werden, wenn der Luftfeuchtewert einen zweiten Schwellwert unterschreitet, und wobei der erste Schwellwert niedriger ist als der zweite Schwellwert,

wobei insbesondere der der erste Schwellwert in einem Bereich von vorzugsweise 0,1 % - 0,4 % unter dem Luftfeuchtesollwert liegt, und
wobei insbesondere der zweite Schwellwert in einem Bereich von vorzugsweise 0,4 % - 3,0% %, unter dem Luftfeuchtesollwert liegt.

7. Steuereinrichtung gemäß einem der vorangehenden Ansprüche, die dazu eingerichtet ist, einen von einem Türsensor eines Inkubators erfassten Türöffnungswert zu erfassen und zu verarbeiten,

und insbesondere die Wasserfördereinrichtung abzuschalten und/oder die Leistung des Heizelements zu reduzieren, wenn gemäß dem Türöffnungswert eine Türe der Inkubatorkammer des Inkubators geöffnet ist, und/oder, wenn gemäß dem Türöffnungswert eine Türe der Inkubatorkammer des Inkubators nach einer Türöffnung wieder geschlossen ist, in einem ersten Betriebsmodus betrieben zu werden, und insbesondere in dem ersten Betriebsmodus die Wasserfördereinrichtung kontinuierlich zu betreiben.

8. Steuereinrichtung gemäß einem der vorangehenden Ansprüche, die dazu eingerichtet ist, in einem dritten Betriebsmodus betrieben zu werden, in dem die Zieltemperatur des Heizelements kleiner als die Verdampfungstemperatur gewählt ist, insbesondere als Temperatur, bei der ein mit dem Heizelement kontaktiertes Wasservolumen nicht verdampft wird.

9. Steuereinrichtung gemäß einem der vorangehenden Ansprüche, die einen in einem Programmcodespeicher gespeicherten Programmcode aufweist, durch dessen Ausführung ein Verdampfungsvorgang, insbesondere durch Definieren des Wasserförderleistungsparameters, gestartet, gestoppt oder beibehalten wird.

10. Steuereinrichtung gemäß einem der vorangehenden Ansprüche, wobei der Verdampfungswert in Abhängigkeit von, insbesondere proportional zu, einer mittleren Leistung des Heizelements bestimmt wird oder in Abhängigkeit von, insbesondere proportional zu, einer Differenz einer aktuellen Heizelementtemperatur von einem Zieltemperatur des Heizelements bestimmt wird.

11. Steuereinrichtung gemäß einem der vorangehenden Ansprüche, wobei ein Programmcode dazu programmiert ist, festzustellen, ob der Verdampfungssollwert von dem Verdampfungswert innerhalb einer vorgegebenen Zeitspanne erreicht wurde, und insbesondere, falls dies nicht der Fall ist, die Wasserfördereinrichtung abzuschalten.

12. Steuereinrichtung gemäß einem der vorangehenden Ansprüche, die einen in einem Programmcodespeicher gespeicherten Programmcode aufweist, der dazu programmiert ist, über eine zeitabhängige Erfassung und Verarbeitung des Verdampfungswertes, der insbesondere durch eine Temperaturänderung oder eine Heizleistungsänderung des Heizelements definiert ist, zu ermitteln, ob ein vorgegebener Schwellwert des Verdampfungswertes, insbesondere innerhalb einer vorbestimmten Zeitdifferenz, überschritten wurde,
und insbesondere die Wasserfördereinrichtung abzuschalten, wenn festgestellt wird, dass ein vorgegebene Schwellwert der Luftfeuchte insbesondere innerhalb einer vorbestimmten Zeitdifferenz überschritten wurde.

13. Steuereinrichtung gemäß einem der vorangehenden Ansprüche, **dadurch gekennzeichnet, dass** die Steuereinrichtung dazu eingerichtet ist, den Luftfeuchtewert zu regeln, indem der Luftfeuchtewert dem Luftfeuchtesollwert asymptotisch angenähert wird, so dass insbesondere ein Überschwingen verhinderbar ist.

14. Verfahren zur elektronischen Steuerung der Befeuchtung der Inkubatoratmosphäre eines Inkubators zur Inkubation lebender Zellkulturen, insbesondere zur Regelung der Luftfeuchte der Inkubatoratmosphäre, mit den Schritten:

- Regeln eines mittels Luftfeuchtesensor messbaren Luftfeuchtewertes der Inkubatoratmosphäre auf einen Luftfeuchtesollwert mittels einer Luftfeuchteregeleinrichtung;
- Regeln einer mittels Temperatursensor messbaren Temperatur eines Heizelements der Verdampfervorrichtung auf eine konstante Zieltemperatur mittels einer Heizregeleinrichtung, wobei die Zieltemperatur als eine Verdampfungstemperatur wählbar ist, die geeignet ist, ein mit dem Heizelement kontaktiertes Wasservolumen zu verdampfen;
- Verwenden eines Wasserförderleistungsparameters zur Ansteuerung einer Wasserfördervorrichtung mittels einer Wasserfördersteuereinrichtung, wobei der Wasserförderleistungsparameters ein aktuell zu förderndes Wasservolumen bestimmt,
- Variieren des Wasserförderleistungsparameter in Abhängigkeit von dem Verdampfungswertes, bis ein mittels Luftfeuchtesensor messbarer Luftfeuchtewert der Inkubatoratmosphäre einem Luftfeuchtesollwert entspricht.

**15.** Verfahren gemäß Anspruch 14, mit den Schritten:

- Regeln eines mittels Luftfeuchtesensor messbaren Luftfeuchtewertes der Inkubatoratmosphäre auf einen vom Inkubator bzw. dem Benutzer vorgegebenen Luftfeuchtesollwert mittels einer Luftfeuchteregeleinrichtung, wobei als Stellgröße ein Verdampfungssollwert verwendet wird,
wobei insbesondere dieser Verdampfungssollwert einem Wasservolumen entspricht, insbesondere zu einem Wasservolumen proportional ist, dessen Verdampfung zur Einstellung der Luftfeuchte der Inkubatoratmosphäre erforderlich ist,
- Regeln eines Verdampfungswertes auf den Verdampfungssollwert mittels einer Wasserförderregeleinrichtung der Verdampfervorrichtung, wobei als Stellgröße ein Wasserförderleistungsparameter einer Wasserfördervorrichtung verwendet wird,
wobei insbesondere dieser Verdampfungswert einem aktuell verdampften Wasservolumen entspricht, insbesondere zu diesem proportional ist, und dieser Wasserförderleistungsparameter einem aktuell geförderten Wasservolumen entspricht, insbesondere zu diesem proportional ist,
- Regeln einer mittels Temperatursensor messbaren Temperatur eines Heizelements der Verdampfervorrichtung auf eine konstante Zieltemperatur mittels einer Heizregeleinrichtung, wobei als Stellgröße der Verdampfungswert verwendet wird,
wobei insbesondere die Zieltemperatur als Verdampfungstemperatur gewählt ist, die geeignet ist, ein während eines Verdampfungsvorgangs mit dem Heizelement in Kontakt kommendes Wasservolumen zu verdampfen und dabei dem Heizelement Wärme zu entziehen, und wobei insbesondere der Verdampfungswert zu einem Wasservolumen proportional ist, das vom Heizelement verdampft wird, und der sich aus einer Messung einer bei dieser Regelung auftretenden Heizleistung oder einer Temperaturabweichung der gemessenen Temperatur von der Zieltemperatur ergibt.

Fig. 1

EP 4 644 523 A1

Fig. 2a

Fig. 2b

Fig. 2c

Fig. 2d

Fig. 3

**Steuereinrichtung** — 100

**103** Wasserförder-steuer-/regel. — 103a
- WP
- w_WP
- WP

**102** Heiz-regeleinrichtung
- x_TC
- w_TC
- y_TC

**101** Luftfeuchte-regeleinrichtung
- x_rH
- w_rH
- y_rH
- 101

**104** Luftentfeuchter-steuereinrichtung

Fig. 4

x_rH = akt. RH Wert

100

Mikrocontroller mit
Verdampfersteuerungsalgorithmus

RH
Sensor

31

Verdampferkammer
2

Dampfauslass

x_TC =
Akt. Verdampfertemperatur

55

51

Shakerkammer

Geregelte Pumpleistung

5

Wasser

Wasserpumpe

Heizelement

Temperatursensor

7

9

Geregelte Heizleistung

Wasser

50

70

Wassertank

EP 4 644 523 A1

29

# Fig. 5

SP = Setpoint/Sollwert
PV = Process Value/Messwert

101

w_rH =
RH setpoint
→ (+ / −) → RH controller → y_rH = w_WP = Vaporizer Heating power (PWM SP) → (+ / −) → Pump controller → y_WP = WP = pump frequency → water pump (5) → steam → Chamber (51) → RH value from RH sensor

103, 103a

filtered PWM values = x_WP ← moving average filter

Direct influence on heating element

102

w_TC =
SP: 180°C → (+ / −) → Vaporizer heater controller → Heater PWM = y_TC → heating element (7)

x_TC = Current heater temperature [°C]

moving average filter

filtered RH value = x_rH

EP 4 644 523 A1

Fig. 6a

RH value vs. pump duty cycle - recovery phase

Fig. 6b

Heater duty cycle vs. RH controller output - recovery phase

## Fig. 7

EP 4 644 523 A1

Fig. 8a

RH value vs. pump duty cycle - fine control (Nachregeln)

t5  t6  t7  t8  t9

RH value [%]    Pump duty cycle [%]

Fig. 8b

Heater duty cycle vs. RH controller output - fine control (Nachregeln)

t5  t6  t7  t8  t9

Vaporizer heater duty cycle [%]    RH Controller output (heater target value) [%]    Vaporizer temperature [°C]

EP 4 644 523 A1

33

# EP 4 644 523 A1

EUROPÄISCHER RECHERCHENBERICHT

Nummer der Anmeldung

EP 24 17 2996

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | DE 10 2017 104508 B3 (ADOLF KUEHNER AG [CH]) 8. März 2018 (2018-03-08) * Absätze [0001], [0003], [0004], [0008], [0009], [0018], [0021], [0023], [0032], [0033] - [0036], [0041] * * Absätze [0044], [0045], [0051], [0057], [0058] * * Abbildung 23 * ----- | 1-15 | INV. C12M1/00 C12M1/36 |
| Y | EP 3 378 928 A1 (BINDER GMBH [DE]) 26. September 2018 (2018-09-26) * Absätze [0005] - [0010], [0017], [0019] - [0021]; Ansprüche 1,4 * ----- | 1-15 | |
| A | EP 3 444 329 A1 (PECON GMBH [DE]) 20. Februar 2019 (2019-02-20) * Absätze [0001], [0003], [0011], [0012], [0018], [0021], [0023]; Ansprüche; Abbildungen * ----- | 1,14 | |
| A | US 4 701 415 A (DUTTON EDMUND L [US] ET AL) 20. Oktober 1987 (1987-10-20) * Spalten 2-3; Ansprüche 1-3 * ----- | 1,14 | RECHERCHIERTE SACHGEBIETE (IPC) C12M |
| A | WO 2022/162056 A1 (EPPENDORF AG [DE]) 4. August 2022 (2022-08-04) * Seiten 9-14 * ----- | 1,14 | |
| A | US 2011/126565 A1 (TSURUMA RYUICHI [JP]) 2. Juni 2011 (2011-06-02) * Absätze [0010], [0011], [0039], [0048] - [0056], [0065], [0066] * ----- -/-- | 1,14 | |

Der vorliegende Recherchenbericht wurde für alle Patentansprüche erstellt

| Recherchenort | Abschlußdatum der Recherche | Prüfer |
|---|---|---|
| Berlin | 15. November 2024 | Böhm, Ingo |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 1 von 2

34

## EUROPÄISCHER RECHERCHENBERICHT

Nummer der Anmeldung

EP 24 17 2996

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 3 626 813 A1 (AIREX CO LTD [JP]) 25. März 2020 (2020-03-25) * Absätze [0001], [0011], [0016], [0019], [0026], [0028], [0032], [0044], [0045]; Ansprüche; Abbildungen * ----- | 1,14 | |
| A | DE 10 2014 106877 A1 (ERT OPTIK DR THIEL GMBH [DE]) 19. November 2015 (2015-11-19) * Absätze [0001], [0015], [0019], [0035], [0036] * ----- | 1,14 | |
| A | EP 2 770 047 B1 (IBIDI GMBH [DE]) 22. April 2015 (2015-04-22) * Abbildungen * ----- | 1,14 | |
| A | DE 10 2004 049210 A1 (SANYO ELECTRIC CO [JP]; SANYO ELECTRIC BIOMEDICAL CO [JP]) 2. Juni 2005 (2005-06-02) * Ansprüche; Abbildungen * ----- | 1,14 | |
| A | DE 44 41 250 C1 (BINDER PETER MICHAEL [DE]) 25. April 1996 (1996-04-25) * Spalten 1,3 * ----- | 1,14 | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| Berlin | 15. November 2024 | Böhm, Ingo |

## ANHANG ZUM EUROPÄISCHEN RECHERCHENBERICHT
## ÜBER DIE EUROPÄISCHE PATENTANMELDUNG NR.

EP 24 17 2996

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-11-2024

| Im Recherchenbericht angeführtes Patentdokument | Datum der Veröffentlichung | Mitglied(er) der Patentfamilie | Datum der Veröffentlichung |
|---|---|---|---|
| DE 102017104508 B3 | 08-03-2018 | CN 108531388 A<br>DE 102017104508 B3<br>EP 3369803 A1<br>US 2018251720 A1 | 14-09-2018<br>08-03-2018<br>05-09-2018<br>06-09-2018 |
| EP 3378928 A1 | 26-09-2018 | CN 108624500 A<br>DE 102017105892 B3<br>EP 3378928 A1<br>PL 3378928 T3<br>RU 2018109418 A<br>US 2018266713 A1 | 09-10-2018<br>21-06-2018<br>26-09-2018<br>07-08-2023<br>16-09-2019<br>20-09-2018 |
| EP 3444329 A1 | 20-02-2019 | KEINE | |
| US 4701415 A | 20-10-1987 | JP S60259178 A<br>US 4701415 A | 21-12-1985<br>20-10-1987 |
| WO 2022162056 A1 | 04-08-2022 | EP 4036206 A1<br>EP 4288516 A1<br>JP 2024503525 A<br>US 2024076602 A1<br>WO 2022162056 A1 | 03-08-2022<br>13-12-2023<br>25-01-2024<br>07-03-2024<br>04-08-2022 |
| US 2011126565 A1 | 02-06-2011 | CN 102080045 A<br>EP 2327759 A1<br>JP 5570191 B2<br>JP 2011110033 A<br>KR 20110060792 A<br>US 2011126565 A1 | 01-06-2011<br>01-06-2011<br>13-08-2014<br>09-06-2011<br>08-06-2011<br>02-06-2011 |
| EP 3626813 A1 | 25-03-2020 | CN 110612346 A<br>EP 3626813 A1<br>JP 6886693 B2<br>JP 2018191546 A<br>KR 20200005538 A<br>US 2020199518 A1<br>WO 2018212029 A1 | 24-12-2019<br>25-03-2020<br>16-06-2021<br>06-12-2018<br>15-01-2020<br>25-06-2020<br>22-11-2018 |
| DE 102014106877 A1 | 19-11-2015 | CN 106536711 A<br>DE 102014106877 A1<br>US 2017073628 A1<br>WO 2015172882 A1 | 22-03-2017<br>19-11-2015<br>16-03-2017<br>19-11-2015 |
| EP 2770047 B1 | 22-04-2015 | CN 104004646 A<br>DK 2770047 T3<br>EP 2770047 A1 | 27-08-2014<br>29-06-2015<br>27-08-2014 |

EPO FORM P0461

Für nähere Einzelheiten zu diesem Anhang : siehe Amtsblatt des Europäischen Patentamts, Nr.12/82

Seite 1 von 2

**ANHANG ZUM EUROPÄISCHEN RECHERCHENBERICHT
ÜBER DIE EUROPÄISCHE PATENTANMELDUNG NR.**

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-11-2024

| Im Recherchenbericht angeführtes Patentdokument | Datum der Veröffentlichung | Mitglied(er) der Patentfamilie | Datum der Veröffentlichung |
|---|---|---|---|
| | | US 2014238496 A1 | 28-08-2014 |
| DE 102004049210 A1 | 02-06-2005 | CN 1616647 A | 18-05-2005 |
| | | DE 102004049210 A1 | 02-06-2005 |
| | | JP 2005118021 A | 12-05-2005 |
| | | KR 20050037959 A | 25-04-2005 |
| | | US 2005084420 A1 | 21-04-2005 |
| DE 4441250 C1 | 25-04-1996 | DE 4441250 C1 | 25-04-1996 |
| | | FR 2727127 A1 | 24-05-1996 |
| | | GB 2295156 A | 22-05-1996 |
| | | JP 2808530 B2 | 08-10-1998 |
| | | JP H08242843 A | 24-09-1996 |
| | | US 5773287 A | 30-06-1998 |

EPO FORM P0461

Für nähere Einzelheiten zu diesem Anhang : siehe Amtsblatt des Europäischen Patentamts, Nr.12/82